(19) Europäisches Patentamt
European Patent Office
Office européen des brevets

(11) **EP 4 524 243 A1**

(12) **EUROPEAN PATENT APPLICATION**
published in accordance with Art. 153(4) EPC

(43) Date of publication:
19.03.2025 Bulletin 2025/12

(21) Application number: 23802929.2

(22) Date of filing: 10.05.2023

(51) International Patent Classification (IPC):
*C12N 7/01* (2006.01)     *A61K 45/00* (2006.01)
*A61P 35/00* (2006.01)

(52) Cooperative Patent Classification (CPC):
A61K 35/761; A61K 35/763; A61K 35/766;
A61K 35/768; A61K 38/17; A61K 45/00;
A61P 35/00; C07K 14/435; C12N 5/10; C12N 7/00;
C12N 15/861; C12R 2001/91

(86) International application number:
PCT/CN2023/093135

(87) International publication number:
WO 2023/217160 (16.11.2023 Gazette 2023/46)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 10.05.2022  CN 202210505579

(71) Applicant: **Neuregen Biotherapeutics (Shanghai)
Co. Ltd
Shanghai 200131 (CN)**

(72) Inventors:
• **LIU, Yueguang
  Shanghai 200131 (CN)**
• **ZHAO, Huihui
  Shanghai 200131 (CN)**

(74) Representative: **Zaboliene, Reda
  Metida
  Business center Vertas
  Gyneju str. 16
  01109 Vilnius (LT)**

Remarks:
The complete document including Reference
Table(s) and the Sequence Listing(s) can be
downloaded from the EPO website

(54) **REPROGRAMMED FUNCTIONAL FRAGMENT OF RECOMBINANT ONCOLYTIC VIRUS, COMBINATION, AND APPLICATION THEREOF**

(57) The present invention provides a reprogrammed functional fragment of a recombinant oncolytic virus, a combination, and an application thereof. The present invention further provides a group of transcription factors and a transcription factor combination which synergistically promote tumor cell transdifferentiation and reprogramming into non-tumor cells, an oncolytic virus expression method which achieves effective synergy of oncolytic therapy and reprogramming effects and can be effectively applied to limiting tumor worsening, and an application of transcription factors carried by the recombinant oncolytic virus in the preparation of a drug for treating tumor diseases.

Fig. 4-2

EP 4 524 243 A1

**Description**

**Background of the Invention**

Field of the Invention

This invention belongs to the fields of biotechnology and gene therapy, specifically relating to the functional fragments, combinations, and applications of reprogrammed recombinant oncolytic viruses. More specifically, it involves a method for transdifferentiating tumor cells into non-tumorigenic cells using an oncolytic virus vector, as well as the application of this method for cancer treatment.

Description of Related Art

One of the key characteristics of tumor cells is uncontrolled cell cycle progression, leading to malignant proliferation. Through reprogramming, tumor cells can be induced to differentiate into non-dividing, non-tumorigenic cells, effectively "correcting" the tumor cells and achieving the goal of cancer treatment. To date, the most successful example of tumor-induced differentiation therapy is the use of all-trans retinoic acid to treat acute promyelocytic leukemia, which has achieved high remission rates.

Oncolytic virus therapy for cancer is gaining increasing attention. The principle involves genetically modifying naturally occurring viruses with low pathogenicity to create specialized oncolytic viruses that selectively infect tumor cells due to the inactivation or defect of tumor suppressor genes in the target cells. These viruses replicate massively inside the tumor cells and ultimately destroy them. At the same time, they trigger an immune response, attracting more immune cells to continue attacking residual cancer cells. Over the past few decades, oncolytic virus therapy has garnered widespread attention, and significant progress has been made in related research. Currently, various oncolytic viruses, including adenoviruses, herpes simplex viruses, coxsackieviruses, poxviruses, polioviruses, measles viruses, and reoviruses, have entered clinical trials. These viruses selectively infect tumor cells, ultimately leading to cell lysis and tumor cell destruction, without replicating in normal cells, theoretically providing higher anti-tumor efficacy and lower side effects.

Oncolytic viruses have now been widely accepted as an important branch of immunotherapy, and their potential in the basic and clinical research of treating malignant tumors has been well established. In 2015, the U.S. Food and Drug Administration and the European Medicines Agency successively approved the HSV-1 oncolytic virus T-VEC for treating melanoma, marking the maturity of oncolytic virus technology and its recognition for treating malignant tumors. Several oncolytic viruses are currently undergoing clinical trials for cancer treatment, with their efficacy and safety being validated.

It is generally believed that oncolytic viruses mediate anti-tumor effects through the following mechanisms: 1) selective replication in tumor cells, directly lysing them; 2) lysed tumor cells release viral particles, triggering systemic anti-tumor immunity through multiple pathways, such as promoting tumor antigen presentation, increasing immune cell infiltration in the tumor microenvironment, modulating the tumor microenvironment, activating immune cells, and activating the immune system through the viral delivery of immune modulators. Additionally, some reports indicate that certain viruses can infect tumor-associated endothelial cells, inhibiting tumor angiogenesis, thereby exerting indirect anti-tumor effects.

Oncolytic adenoviruses are currently the most commonly used oncolytic viruses, and various strategies have been developed to enhance their tumor-targeting abilities. These strategies include mutating functional genes involved in cell cycle control (such as E1A or E1B) in the adenoviral genome, using tumor-specific promoters to regulate the targeted transcription of the E1A gene, employing different adenoviral serotypes or RGD motifs to alter the targeting transduction pathways by which the oncolytic adenovirus enters tumor cells, and using cellular carriers to deliver the oncolytic adenovirus to distant tumor sites. Oncolytic adenoviruses can also serve as vectors to deliver immune-modulating genes or therapeutic genes, generating synergistic anti-tumor effects by enhancing anti-tumor immunity or inducing tumor cell apoptosis or suicide. Currently, oncolytic viruses often carry immune-modulating factors or suicide genes, further enhancing tumor treatment outcomes, though there are still challenges with efficacy.

Gliomas, also known as glial cell tumors, are one of the most lethal malignant tumors and the most common primary tumors of the central nervous system, accounting for 30% of brain and central nervous system tumors and 80% of malignant brain tumors. Gliomas pose a serious threat to human health. According to the 1999 World Health Organization (WHO) classification, gliomas include astrocytomas, oligodendrogliomas, ependymomas, mixed gliomas, choroid plexus tumors, neuroepithelial tumors of uncertain origin, neuronal and neuronal-glial mixed tumors, pineal parenchymal tumors, embryonal tumors, and neuroblastomas. Gliomas interweave with normal neural tissue, making it difficult to define their boundaries, and they tend to recur. Additionally, due to the blood-brain barrier, conventional anti-tumor drugs are often ineffective. The treatment of gliomas remains an unmet clinical need in the medical field. In recent years, some studies have shown that certain neurogenic transcription factors or combinations of transcription factors can reprogram glioma cells into neuron-like cells, thereby limiting their proliferative capacity. However, common delivery vectors, such as adeno-associated virus (AAV) vectors, cannot replicate and are unable to transfer foreign genes into a sufficient number of tumor cells, limiting their clinical utility for cancer therapy.

Therefore, finding and utilizing suitable replicative expression vectors to deliver appropriate reprogramming factors, infect tumor cells, and replicate within them-leading to the release of the virus to infect more tumor cells-is a new therapeutic approach for cancer treatment. In this process, some tumor cells are lysed and killed, some die due to anti-tumor immunity, and others are reprogrammed into non-dividing, non-tumorigenic cells, achieving effective synergy in cancer treatment.

## Summary of the Invention

The objective of the present invention is to address the shortcomings of the prior art by providing a reprogramming functional fragment of a recombinant oncolytic virus, a combination thereof, and its applications. The invention offers a set of transcription factors and transcription factor combinations that synergistically promote tumor cell trans-differentiation and reprogramming into non-tumorigenic cells, utilizing a method in which these transcription factors are expressed through an oncolytic virus, and the application of these oncolytic virus-carried transcription factors in the preparation of drugs for tumor diseases.

To achieve this objective, the invention adopts the following technical solution:

In the first aspect of the present invention, a recombinant oncolytic virus is provided, wherein the recombinant oncolytic virus comprises recombinant nucleic acid, the recombinant nucleic acid comprising a functional fragment that promotes reprogramming/trans-differentiation of tumor cells into non-tumorigenic cells;

The functional fragment comprises at least one functional fragment that promotes the expression of a transcription factor, wherein the functional fragment is selected from a functional fragment that promotes the expression of at least one transcription factor from NeuroD1, Brn2, Ascll, or Ngn2.

In another preferred embodiment, the functional segment that promotes transcription factor expression contained in the recombinant oncolytic virus includes at least the functional segment that promotes the expression of the Ascll transcription factor.

In another preferred embodiment, the functional segment that promotes transcription factor expression contained in the recombinant oncolytic virus includes at least the functional segment that promotes the expression of the NeuroD1 transcription factor.

In another preferred embodiment, the functional segment that promotes transcription factor expression contained in the recombinant oncolytic virus includes at least the functional segment that promotes the expression of the Brn2 transcription factor.

In another preferred embodiment, the functional segment that promotes transcription factor expression contained in the recombinant oncolytic virus includes at least the functional segment that promotes the expression of the Ngn2 transcription factor.

In another preferred embodiment, the recombinant nucleic acid comprises a set of functional fragments that synergistically promote reprogramming/trans-differentiation of tumor cells into non-tumorigenic cells. The functional fragment comprises at least two functional fragments that promote the expression of transcription factors, wherein the functional fragments are selected from functional fragments that promote the expression of at least two transcription factors from NeuroD1, Brn2, Ascll, or Ngn2.

In another preferred embodiment, the functional segment promoting transcription factor expression contained in the recombinant oncolytic virus includes at least the functional segment that promotes the expression of the NeuroD1 transcription factor and another functional segment that promotes transcription factor expression, wherein the other functional segment is selected from any functional segment that promotes the expression of transcription factors such as Ascll, Ngn2, or Brn2.

More preferably, the other functional segment that promotes transcription factor expression is selected from any functional segment that promotes the expression of transcription factors such as Ascll or Ngn2.

In another preferred embodiment, the functional segment promoting transcription factor expression contained in the recombinant oncolytic virus includes at least the functional segment that promotes the expression of the Ngn2 transcription factor and another functional segment that promotes transcription factor expression, wherein the other functional segment is selected from any functional segment that promotes the expression of transcription factors such as NeuroD1, Brn2, or Ascll.

More preferably, the other functional segment that promotes transcription factor expression is selected from any functional segment that promotes the expression of transcription factors such as Ascll or NeuroD1. Further preferably, the other functional segment that promotes transcription factor expression is a functional segment that promotes the expression of the Ascll transcription factor, i.e., the recombinant oncolytic virus contains recombinant nucleic acid, and the recombinant nucleic acid includes functional segments that promote tumor cell reprogramming/trans-differentiation, wherein the functional segments simultaneously contain functional segments that promote the expression of the Ascll and Ngn2 transcription factors.

In another preferred embodiment, the functional segments contained in the recombinant oncolytic virus that can synergistically promote tumor cell reprogramming/trans-differentiation or promote transcription factor expression are polynucleotides encoding functional proteins, and the functional proteins are transcription factors such as functional NeuroD1, Brn2, Ascll, and Ngn2.

Preferably, the functional segments contained in the oncolytic virus that can synergistically promote glial cell reprogramming/trans-differentiation or promote transcription factor expression are derived from mammals, more preferably, the functional segments are derived from humans or non-human primates.

In another preferred embodiment, the functional segment contained in the recombinant oncolytic virus, which can synergistically promote tumor cell reprogramming/trans-differentiation, is a polynucleotide encoding a functional protein, and the functional protein is the functional NeuroD1 protein, the amino acid sequence of which is shown in SEQ ID NO.1 or SEQ ID NO.2; the polynucleotide sequence encoding the functional NeuroD1 protein is shown in SEQ **ID** NO.3 or SEQ **ID** NO.4.

In another preferred embodiment, the functional segment contained in the recombinant oncolytic virus, which can synergistically promote tumor cell trans-differentiation, is a polynucleotide encoding a functional protein, and the functional protein is the functional Brn2 protein, the amino acid sequence of which is shown in SEQ ID NO.5 or SEQ ID NO.6; the polynucleotide sequence encoding the functional Brn2 protein is shown in SEQ ID NO.7 or SEQ ID NO.8.

In another preferred embodiment, the functional segment contained in the recombinant oncolytic virus, which can synergistically promote tumor cell trans-differentiation, is a polynucleotide encoding a functional protein, and the functional protein is the functional Ascll protein, the amino acid sequence of which is shown in SEQ ID NO.9, SEQ ID NO.10, or SEQ ID NO.18; the polynucleotide sequence encoding the functional Ascl1 protein is shown in SEQ ID NO.11, SEQ ID NO.12, or SEQ ID NO.19.

In another preferred embodiment, the functional segment contained in the recombinant oncolytic virus, which can synergistically promote tumor cell trans-differentiation, is a polynucleotide encoding a functional protein, and the functional protein is the functional Ngn2 protein, the amino acid sequence of which is shown in SEQ ID NO. 13 or SEQ ID NO.14; the polynucleotide sequence encoding the functional Ngn2 protein is shown in SEQ ID NO.15 or SEQ ID NO.16.

In another preferred embodiment, when the functional segment contained in the recombinant oncolytic virus, which can synergistically promote tumor cell trans-differentiation, is a polynucleotide encoding a functional protein, the amino acid sequence of the functional protein has a sequence identity of not less than 85% with the sequence of SEQ ID NO.1, SEQ ID NO.2, SEQ ID NO.5, SEQ ID NO.6, SEQ ID NO.9, SEQ ID NO.10, SEQ ID NO.13, SEQ ID NO.14, or SEQ ID NO.18, for example, 85%, 87%, 89%, 90%, 91%, 93%, 95%, 97%, or 99%.

More preferably, the amino acid sequence of the functional protein has a sequence identity of not less than 95% with the sequence of SEQ ID NO.1, SEQ ID NO.2, SEQ ID NO.5, SEQ ID NO.6, SEQ ID NO.9, SEQ ID NO.10, SEQ ID NO.13, SEQ ID NO.14, or SEQ ID NO.18, for example, 95%, 96%, 97%, 98%, or 99%.

Most preferably, the amino acid sequence of the functional protein has a sequence identity of not less than 99% with the sequence of SEQ ID NO.1, SEQ ID NO.2, SEQ ID NO.5, SEQ ID NO.6, SEQ ID NO.9, SEQ ID NO.10, SEQ ID NO.13, SEQ ID NO.14, or SEQ ID NO.18.

In another preferred embodiment, when the functional segment contained in the recombinant oncolytic virus, which can synergistically promote tumor cell trans-differentiation, is a polynucleotide encoding a functional protein, the polynucleotide sequence encoding the functional protein has a sequence identity of not less than 75% with the sequence of SEQ ID NO.3, SEQ ID NO.4, SEQ ID NO.7, SEQ ID NO.8, SEQ ID NO.11, SEQ ID NO.12, SEQ ID NO.15, SEQ ID NO.16, or SEQ ID NO.19, for example, 75%, 77%, 79%, 80%, 85%, 87%, 89%, 90%, 91%, 93%, 95%, 97%, or 99%.

More preferably, the polynucleotide sequence encoding the functional protein has a sequence identity of not less than 85% with the sequence of SEQ ID NO.3, SEQ ID NO.4, SEQ ID NO.7, SEQ ID NO.8, SEQ ID NO.11, SEQ ID NO.12, SEQ ID NO.15, SEQ ID NO.16, or SEQ ID NO.19, for example, 85%, 87%, 89%, 90%, 91%, 93%, 95%, 97%, or 99%.

Most preferably, the polynucleotide sequence encoding the functional protein has a sequence identity of not less than 95% with the sequence of SEQ ID NO.3, SEQ ID NO.4, SEQ ID NO.7, SEQ ID NO.8, SEQ ID NO.11, SEQ ID NO.12, SEQ ID NO.15, SEQ ID NO.16, or SEQ ID NO.19, for example, 95%, 96%, 97%, 98%, or 99%.

Preferably, the expression system for the functional fragments promoting the expression of transcription factors is constructed under the same expression vector or is expressed using different expression vectors, respectively.

Preferably, the recombinant oncolytic virus includes a selectively replicating recombinant oncolytic virus.

Preferably, the selectively replicating recombinant oncolytic virus is derived from an oncolytic adenovirus, poxvirus, herpes simplex virus, measles virus, Semliki Forest virus, vesicular stomatitis virus, poliovirus, retrovirus, reovirus, Seneca Valley virus, echovirus, coxsackievirus, Newcastle disease virus, or Maraba virus.

Preferably, the tumor cells are any type of glioblastoma, glioma, astrocytoma, astroblastoma, medulloblastoma, schwannoma, brain metastases, neuroblastoma, chordoma, meningioma, teratoma, spinal tumor, breast cancer, head and neck tumors, kidney cancer, melanoma, lung cancer, esophageal cancer, colon cancer, rectal cancer, brain cancer, liver cancer, bone cancer, choriocarcinoma, gastrinoma, pheochromocytoma, prolactinoma, cholangiocarcinoma, bladder cancer, ureteral cancer, glioma, osteochondroma, chondrosarcoma, Ewing's sarcoma, fibrosarcoma, cervical cancer, gallbladder cancer, eye cancer, Kaposi's sarcoma, prostate cancer, testicular cancer, squamous cell carcinoma of the skin, mesothelioma, ovarian cancer, pancreatic neuroendocrine tumors, glucagonoma, pancreatic cancer, pituitary cancer, soft tissue sarcoma, retinoblastoma, small intestine cancer, gastric cancer, thymic cancer, trophoblastic cancer, endometrial cancer, vaginal cancer, vulvar cancer, insulinoma, blood cancer, peritoneal cancer, or pleural cancer from humans or non-human mammals.

Preferably, the reprogramming/trans-differentiation refers to reprogramming or transdifferentiating tumor cells into non-tumorigenic cells.

In the second aspect of this invention, a method is provided for promoting the reprogramming/trans-differentiation of tumor cells into non-tumorigenic cells through oncolytic viruses. This method includes the following steps: contacting the recombinant oncolytic virus described in the first aspect of the invention with tumor cells, thereby enabling the tumor cells to be reprogrammed/transdifferentiated into non-tumorigenic cells.

In another preferred embodiment, the method is non-therapeutic and non-diagnostic.

In another preferred embodiment, the method is an in vitro method.

In another preferred embodiment, the method is an in vivo method.

In another preferred embodiment, the method is therapeutic. It should be noted that this method does not conflict with existing tumor therapies and can be used in combination with treatments such as immunotherapy, CAR-T, and tumor-treating fields (TTF).

Preferably, the tumor cells include any glioblastoma, glioma, astrocytoma, astroblastoma, medulloblastoma, Schwannoma, brain metastasis, neuroblastoma, chordoma, meningioma, teratoma, spinal cord tumor, breast cancer, head and neck tumor, kidney cancer, melanoma, lung cancer, esophageal cancer, colon cancer, rectal cancer, brain cancer, liver cancer, bone cancer, choriocarcinoma, gastrinoma, pheochromocytoma, prolactinoma, cholangiocarcinoma, bladder cancer, ureteral cancer, glioma, osteochondroma, chondrosarcoma, Ewing's sarcoma, fibrosarcoma, cervical cancer, gallbladder cancer, eye cancer, Kaposi's sarcoma, prostate cancer, testicular cancer, skin squamous cell carcinoma, mesothelioma, ovarian cancer, pancreatic neuroendocrine tumor, glucagonoma, pancreatic cancer, pituitary cancer, soft tissue sarcoma, retinoblastoma, small intestine cancer, stomach cancer, thymic cancer, trophoblastic cancer, endometrial cancer, vaginal cancer, vulvar cancer, insulinoma, blood cancer, peritoneal cancer, or pleural cancer derived from any human or non-human mammal.

In another preferred embodiment, any method that promotes the increased expression of transcription factors that facilitate glial cell trans-differentiation, including but not limited to direct contact or introduction of induction factors or functional fragments that promote the expression of transcription factors, can be used to enhance the expression of any one of the NeuroD1, Brn2, Ascll, or Ngn2 transcription factors in glial cells, thereby promoting the trans-differentiation of the tumor cells into non-tumorigenic cells.

In this invention, the delivery system comprises the oncolytic virus, with the selectively replicating recombinant oncolytic virus being derived from oncolytic adenoviruses, poxviruses, herpes simplex viruses, measles viruses, Semliki Forest viruses, vesicular stomatitis viruses, polioviruses, retroviruses, reoviruses, Seneca Valley viruses, echoviruses, coxsackieviruses, Newcastle disease viruses, or Maraba viruses.

In another preferred embodiment, the recombinant oncolytic virus carrying the expression vector for transcription factor polynucleotides may also carry other functional fragments, which can include reporter genes or functional fragments of other transcription factors involved in reprogramming, including but not limited to NeuroD1, Brn2, Ascll, or Ngn2.

Preferably, at least two polynucleotide fragments of transcription factors can be included in the same vector, where these two transcription factor polynucleotide fragments can be expressed under one tumor cell-specific promoter or under two separate tumor cell-specific promoters. When two or more transcription factors are within a single promoter's transcript, the promoter is linked to the open reading frames of multiple transcription factors through a polycistronic element, with transcription factors being spaced apart using elements like IRES or 2A peptide (P2A) to facilitate the expression of multiple transcription factors.

In another preferred embodiment, the recombinant oncolytic virus contains a set of functional fragments that synergistically promote the reprogramming/trans-differentiation of tumor cells. The expression system for functional fragments that promote transcription factor expression can be constructed under the same expression vector or expressed separately using different expression vectors.

In the third aspect of this invention, a composition for treating cancer is provided, which includes:

(A) a recombinant oncolytic virus as described in the first aspect;
(B) a pharmaceutically acceptable excipient.

In some embodiments, the composition further includes:
(C) an anti-tumor drug;
The anti-tumor drug includes one or both of temozolomide and bevacizumab.

In another preferred embodiment, the pharmaceutical composition is a liquid formulation or a lyophilized formulation.

In another preferred embodiment, the pharmaceutical composition is an injectable formulation.

In the fourth aspect of this invention, the recombinant oncolytic virus as described in the first aspect is provided for use in the preparation of a medicament for treating tumors.

In another preferred embodiment, the recombinant oncolytic virus is formulated as a therapeutic agent that is administered through a site within or near the tumor, and the administration method of the therapeutic agent includes one or more of hydrogel delivery, convection-enhanced delivery and Ommaya reservoir delivery.

Preferably, the tumor comprises: glioblastoma, neuroblastoma, chordoma, meningioma, teratoma, spinal cord tumor, breast cancer, head and neck tumor, renal cancer, melanoma, lung cancer, esophageal cancer, colon cancer, rectal cancer, brain cancer, liver cancer, bone cancer, choriocarcinoma, gastrinoma, pheochromocytoma, prolactinoma, cholangiocarcinoma, bladder cancer, ureter cancer, glioma, osteochondroma, chondrosarcoma, Ewing's sarcoma, fibrosarcoma, cervical cancer, gallbladder cancer, eye cancer, Kaposi's sarcoma, prostate cancer, testicular cancer, squamous cell carcinoma of the skin, mesothelioma, ovarian cancer, pancreatic neuroendocrine tumor, glucagonoma, pancreatic cancer, pituitary carcinoma, soft tissue sarcoma, retinoblastoma, small intestine cancer, gastric cancer, thymic cancer, trophoblastic cancer, endometrial cancer, vaginal cancer, vulvar cancer, insulinoma, hematological cancer, peritoneal cancer, or pleural cancer.

The numerical ranges described in this invention not only include the explicitly listed specific values but also encompass any arbitrary specific values within the unlisted ranges. For brevity and clarity, this invention does not exhaustively enumerate the specific values included within the stated ranges.

**Brief Description of the Figures**

Figures 1A and 1B show the cytotoxicity curves of different infection multiplicities of the Ad5-AN oncolytic adenovirus against U87 and U118 cells in Example 2.

Figures 2A and 2B demonstrate that Ad5-AN can transdifferentiate glioma cells U251 into non-tumorigenic neuronal cells in Example 3.

Figures 3A, 3B, and 3C illustrate that the oncolytic adenoviral vector expressing reprogramming factors inhibits the growth of tumor cells in a mouse model of ectopic implantation of glioma in Example 4; Figure 3A-2 presents the experimental results after extending the duration of the experiment in Figure 3A.

Figure 4 displays the results of a combination therapy experiment with the oncolytic virus expressing reprogramming factors in Example 5; Figure 4-2 shows the experimental results after extending the duration of the experiment in Figure 4.

Figure 5 shows the results of seven parallel experiments in the temozolomide (TMZ) group in Example 5, where all seven mice numbered A-G experienced tumor recurrence after 56 days of treatment (7/7).

Figure 6 presents the results of seven parallel experiments in the combination therapy group of Ad5-AN and temozolomide in Example 5, where none of the seven mice numbered A-G experienced tumor recurrence after 93 days of treatment (0/7).

**Detailed Description of the Invention**

Through extensive and in-depth research, the inventors of this invention have discovered that utilizing an oncolytic virus expression vector to carry a set of transcription factors or combinations of transcription factors with trans-differentiation reprogramming functions can differentiate tumor cells into non-dividing, non-tumorigenic cells, both in vitro and in vivo. Based on this finding, the inventors further explored the application of this method in the development of cancer drugs, particularly in glioma animal models, and observed that the oncolytic virus expression vector can achieve an effective synergy of oncolytic therapy and reprogramming effects, enhancing anti-tumor efficacy. The tumor size in the animals significantly decreased, and their survival time was markedly prolonged. Therefore, this set of transcription factors or combinations of transcription factors with trans-differentiation/reprogramming functions utilizing oncolytic virus expression vectors holds promise for application in the development of cancer drugs, especially glioma drugs.

Terminology

The term "administration" refers to any method and delivery system known to those skilled in the art for physically introducing the products of this invention into a subject, including intravenous, intracranial, intratumoral, intramuscular, subcutaneous, intraperitoneal, spinal, or other extragastrointestinal routes of administration, such as through injection or infusion.

The term "about" can refer to values or compositions within an acceptable margin of error for specific values or compositions determined by those skilled in the art, which will partially depend on how the values or compositions are measured or determined. Generally, "about" indicates $\pm$ 10% or $\pm$20%. For example, about 1:1 means $(1 \pm 0.2):(1 \pm 0.2)$ or $(1 \pm 0.1):(1 \pm 0.1)$. As used herein, the term "reprogramming" generally refers to the process of regulating or altering the biological activity of a cell, transforming it from one biological state to another, typically including processes such as differentiation (from progenitor cells to terminal cells), dedifferentiation (from terminal cells to pluripotent stem cells), trans-differentiation (from one terminal cell to another terminal cell), redifferentiation (from terminal cells to progenitor cells), and trans-determination (from one progenitor cell to another progenitor cell's naturally differentiated terminal cell), among others that change cell fate.

In this invention, "trans-differentiation" or "reprogramming" or "trans-differentiation reprogramming" or "trans-differentiation/reprogramming" or "reprogramming/trans-differentiation" specifically refers to the process of changing one terminal cell to another terminal cell, particularly the process of converting tumor cells into non-tumorigenic cells.

**Transcription Factors**

The present invention provides a set of transcription factors with reprogramming functions, which possess excellent trans-differentiation abilities and can be used to promote the efficiency of glial cell trans-differentiation into neurons.

As used herein, the term "transcription factors of the present invention" refers to one or a group of transcription factors necessary for neural cell differentiation, selected from the following group: NeuroD1, Brn2, Ascll, Ngn2, Gsx1, Tbr1, Dlx2, Ptfla, Pax6, and Otx2.

Preferably, the transcription factors of the present invention include at least two of the aforementioned transcription factors.

The functional fragment of NeuroD1 is a polynucleotide derived from mammals that encodes the Neurogenic differentiation 1 transcription factor or its expressed protein fragment. NeuroD1 is a bHLH (basic helix-loop-helix) transcription factor. For instance, the human NeuroD1 molecule has the GenBank ID# 4760, and its protein sequence is shown as SEQ ID NO.1; the NCBI Reference Sequence is NM_002500.5, with the CDS sequence shown as SEQ ID NO.3.

The functional fragment of Brn2, also known as POU3F2, Oct7, or N-Oct3, is a polynucleotide derived from mammals that encodes the Pou class 3 homeobox 2 transcription factor or its expressed protein fragment. Brn2 is a neural cell-specific POU-III-type transcription factor family. For example, the human Brn2 molecule has the GenBank ID# 5454, and its protein sequence is shown as SEQ ID NO.5; the NCBI Reference Sequence is NM_005604.4, with the CDS sequence shown as SEQ ID NO.7.

The functional fragment of Ascl1 is a polynucleotide derived from mammals that encodes the Achaete-scute homolog 1 transcription factor or its expressed protein fragment. Ascll is a bHLH (basic helix-loop-helix) transcription factor. For example, the human Ascl1 molecule has the GenBank ID# 429, and its protein sequence is shown as SEQ ID NO.9; the NCBI Reference Sequence is NM_004316.4, with the CDS sequence shown as SEQ ID NO.11.

The functional fragment of Ngn2, also known as Neurog2, is a polynucleotide derived from mammals that encodes the Neurogenin-2 transcription factor or its expressed protein fragment. Ngn2 is a bHLH (basic helix-loop-helix) transcription factor. For instance, the human Ngn2 molecule has the GenBank ID# 63973, and its protein sequence is shown as SEQ ID NO. 13; the NCBI Reference Sequence is NM_024019.4, with the CDS sequence shown as SEQ ID NO.15.

Any method that can promote the increased expression of transcription factors facilitating the trans-differentiation of glial cells includes, but is not limited to, introducing functional fragments of inductive factors or promoting transcription factor expression through direct contact with the tumor cells or through introduction, thereby enhancing the expression of any of the NeuroD1, Brn2, Ascll, and Ngn2 transcription factors in glial cells, promoting the trans-differentiation of the tumor cells into non-tumorigenic cells. The methods for promoting the expression of the aforementioned functional fragments of transcription factors may also include targeting the DNA activation genes of related transcription factors through CRISPR/dCas9 to enhance their expression or improving the expression of functional transcription factor proteins by targeting the RNA of related transcription factors through CRISPR/Cas 13.

Those skilled in the art can screen the methods for promoting the above transcription factors based on existing databases. It should be understood that based on the functional trans-differentiation of tumor cells disclosed in this invention, those skilled in the art can reasonably anticipate that any substances that promote the above transcription factors will function in the trans-differentiation of tumor cells.

Preferably, the transcription factors with reprogramming functions of the present invention can be used in conjunction with modified expression elements to further enhance the expression of the transcription factors of the present invention.

## Oncolytic Virus

The oncolytic virus described in this invention is a replicative recombinant oncolytic virus derived from adenovirus, vaccinia virus, herpes simplex virus, measles virus, Semliki Forest virus, vesicular stomatitis virus, poliovirus, retrovirus, enterovirus, Seneca Valley virus, echovirus, coxsackievirus, Newcastle disease virus, and Maraba virus.

The oncolytic virus that can be used in this invention is not specifically limited; preferably, it is derived from type 5 adenovirus, vaccinia virus, and type 1 or type 2 herpes simplex virus.

## Tumor Cells

As used herein, tumor cells refer to any glioblastoma, glioma, astrocytoma, astrocytoblastoma, medulloblastoma, schwannoma, brain metastases, neuroblastoma, chordoma, meningioma, teratoma, spinal tumor, breast cancer, head and neck tumors, kidney cancer, melanoma, lung cancer, esophageal cancer, colon cancer, rectal cancer, brain cancer, liver cancer, bone cancer, choriocarcinoma, gastrinoma, pheochromocytoma, prolactinoma, cholangiocarcinoma, bladder cancer, ureter cancer, neuroglial tumors, osteosarcoma, chondrosarcoma, Ewing's sarcoma, fibrosarcoma, cervical cancer, gallbladder cancer, eye cancer, Kaposi's sarcoma, prostate cancer, testicular cancer, squamous cell carcinoma of the skin, mesothelioma, ovarian cancer, pancreatic endocrine tumors, glucagonoma, pancreatic cancer, pituitary cancer, soft tissue sarcoma, retinoblastoma, small intestine cancer, gastric cancer, thymic cancer, trophoblastic tumors, endometrial cancer, vaginal cancer, vulvar cancer, insulinoma, hematological cancers, peritoneal cancer, or pleural cancer derived from human or non-human mammals.

There are no specific restrictions on the tumor cells that can be used in this invention; preferably, they are derived from various gliomas originating from the mammalian central nervous system, such as glioblastoma, astrocytoma, oligodendroglioma, ependymoma, or neuroblastoma.

Neuroglial tumors, commonly referred to as gliomas or glioblastomas, broadly refer to all tumors of neuroepithelial origin, specifically referring to tumors derived from various glial cells. Gliomas are among the deadliest malignant tumors and are the most common primary central nervous system tumors, accounting for 30% of brain and central nervous system tumors and 80% of malignant brain tumors, posing a significant threat to human health. According to the World Health Organization (WHO) classification scheme from 1999, they are divided into astrocytoma, oligodendroglioma, ependymoma, mixed glioma, choroid plexus tumors, neuroepithelial tumors, neuronal and neuroglial mixed tumors, pineoblastoma, embryonal tumors, and neuroblastoma.

## Pharmaceutical Composition and Administration Methods

This invention also provides a pharmaceutical composition, which is a drug composition for cancer treatment, comprising a recombinant oncolytic virus that contains functional fragments promoting the expression of tumor cell transdifferentiation transcription factors and a pharmaceutically acceptable carrier.

A pharmaceutically acceptable carrier refers to carriers used for drug delivery, including various excipients and diluents.

The pharmaceutical composition of this invention typically contains $10^6$-$10^9$ PFU/g of type 5 adenovirus particles, preferably $10^6$-$10^8$ PFU/g of type 5 adenovirus particles, and more preferably $10^7$-$10^8$ PFU/g of type 5 adenovirus particles.

In the pharmaceutical composition of this invention, it typically contains $10^5$-$10^8$ PFU/g of type 1 herpes simplex virus particles, preferably $10^6$-$10^8$ PFU/g of type 1 herpes simplex virus particles, and more preferably $10^6$-$10^7$ PFU/g of type 1 herpes simplex virus particles.

Pharmaceutically acceptable carriers may include any one or at least a combination of coating layers, capsules, microcapsules, or nanocapsules. It should be noted that the carrier needs to be non-toxic and should not significantly affect the activity of key components in the composition (such as the above-mentioned oncolytic virus and anti-cancer promoting molecules expressed by the oncolytic virus). In some embodiments, the carrier can protect key components in the composition, reducing or avoiding inactivation or degradation of key components under certain adverse conditions (such as denaturation caused by oxidation, strong acids, or strong bases). For example, enzymes in gastric fluid or relatively low pH may lead to degradation or inactivation of key components. The carrier can help maintain or enhance the efficacy of the pharmaceutical composition by protecting key components within the composition.

In some embodiments, the carrier may be used for the controlled release of key components (such as the oncolytic virus). Controlled release may include but is not limited to slow release, sustained release, or targeted release. For example, the carrier may include hydrogels, microcapsules, or nanocapsules made from any one or at least a combination of collagen, gelatin, chitosan, alginate, polyethylene glycol, polyethylenimine, starch, or crosslinked starch.

In some embodiments, pharmaceutically acceptable carriers may include dispersion media (such as solvents), coatings, buffers, stabilizers, isotonic agents, or absorption delay agents. Exemplary pharmaceutically acceptable carriers may include phosphate buffered saline solution, water, emulsions (e.g., oil/water emulsions), various types of wetting agents, sterile solutions, gels, or biocompatible matrix materials, or other suitable materials, or any combination thereof.

In some embodiments, excipients may include but are not limited to water, saline, polyethylene glycol, hyaluronic acid, ethanol, or pharmaceutically acceptable salts, such as salts of inorganic acids (e.g., hydrochloric acid, hydrobromic acid, phosphoric acid, or sulfuric acid) or salts of organic acids (e.g., acetate, propionate, or benzoate).

Typically, after mixing the expression vector with the pharmaceutically acceptable carrier, the pharmaceutical composition of this invention can be obtained.

There are no specific restrictions on the administration methods of the compositions described in this invention. Representative examples include (but are not limited to): injection at the tumor site or nearby, hydrogel delivery, convection-enhanced delivery (CED), Ommaya reservoir delivery, intraperitoneal delivery, subarachnoid delivery, or intravenous delivery.

The pharmaceutical composition can be applied to subjects with cancer, such as humans or animals. In some embodiments, the pharmaceutical composition may be administered to subjects by one or more administration methods. These one or more administration methods may include but are not limited to oral, injection, or topical administration. Suitable forms for oral administration can include but are not limited to tablets, liposome preparations, sustained-release capsules, microparticles, microspheres, or any other suitable forms. Suitable forms for injectable administration can include but are not limited to sterile aqueous solutions or oily preparations. Suitable forms for topical administration can include but are not limited to sterile aqueous solutions or non-aqueous solutions, suspensions, or emulsions. For nasal administration, suitable forms may include aerosols, mists, powders, solutions, suspensions, or gels.

In some embodiments, the pharmaceutical composition can be stored at suitable temperatures, including room temperature (approximately 20° C), 40° C, -20° C, and -80° C, etc. The composition can also be formulated into various forms conducive to storage and transport, such as powders. Powders may be sterile powders, which can be reconstituted by adding a solvent before use to prepare a solution for oral, injectable, or topical administration. In some embodiments, the pharmaceutical composition may also include components that have antibacterial effects but do not have a significant negative impact on the survival of the oncolytic virus, ensuring that the pharmaceutical composition is stable under certain storage conditions (e.g., refrigeration and freezing) and can prevent contamination by microorganisms (such as bacteria and fungi).

**Therapeutic Applications**

The recombinant oncolytic virus described in this invention contains any molecular entity that promotes the expression or activity enhancement of the functional fragment of the transcription factor or a delivery system containing the functional fragment or activity-enhancing molecular entity for preparing anti-tumor drugs.

The compositions disclosed in this application may be used before or after the administration of other drug compositions for treating cancer. Alternatively, the compositions disclosed in this application can be combined with other therapeutic methods to treat the cancer afflicting the subject. For example, other therapeutic methods may include but are not limited to administering other drug compositions that can treat cancer, surgically removing tumors from the subject, radiotherapy, or electrotherapy. Specifically, drug compositions for treating cancer may include but are not limited to cytotoxic anti-cancer drugs and/or non-cytotoxic anti-cancer drugs. Non-cytotoxic anti-cancer drugs may include hormonal drugs, targeted drugs (e.g., bevacizumab), or immunotherapy drugs (e.g., monoclonal antibodies and/or tumor vaccines).

Beneficial Effects of the Invention

Through extensive and in-depth research, the inventors have discovered that using oncolytic virus expression vectors carrying a set of transcription factors or combinations of transcription factors with transdifferentiation and reprogramming functions can differentiate tumor cells into non-tumorigenic cells that no longer divide, either in vitro or in vivo. Based on this discovery, the inventors further explored the application of this method in tumor drug development, particularly in glioma animal models, where it was observed that the oncolytic virus expression vectors could achieve effective synergy between oncolytic therapy and reprogramming effects, enhancing the anti-tumor effect. Tumor sizes in the animals were significantly reduced, and survival times were notably extended. Therefore, this set of transcription factors or combinations with trans-differentiation/reprogramming functions, delivered through oncolytic virus expression vectors, holds promise for application in the development of tumor drugs, especially glioma therapies.

Compared to the prior art, this invention has the following competitive advantages: innovatively combining oncolytic viruses with reprogramming to produce a set of reprogramming transcription factors or combinations expressed in oncolytic viruses, as well as recombinant oncolytic viruses. The ability of these transcription factors and combinations to transdifferentiate tumor cells into non-tumorigenic cells has been explored. Such recombinant oncolytic viruses exhibit both high tumor-killing efficiency and tumor suppression, offering improved tumor treatment and preventing recurrence.

The following specific embodiments further illustrate the technical solutions of this invention. Those skilled in the art should understand that the examples are merely intended to aid in understanding the invention and should not be regarded as limiting the scope of the invention.

Unless otherwise specified, experimental methods in the following examples are conducted under standard conditions, such as those described by Sambrook et al. in *Molecular Cloning: A Laboratory Manual* (New York: Cold Spring Harbor Laboratory Press, 1989), or according to the manufacturer's recommended conditions.

**General Methods**

**Materials and Methods**

**Amino Acid Sequence**

SEQ ID NO.1 (hNeuroD1 Amino Acid Sequence):

MTKSYSESGLMGEPQPQGPPSWTDECLSSQDEEHEADKKEDDLETMNAEEDSL
RNGGEEEDEDEDLEEEEEEEEEDDDQKPKRRGPKKKKMTKARLERFKLRRMKANAR
ERNRMHGLNAALDNLRKVVPCYSKTQKLSKIETLRLAKNYIWALSEILRSGKSPDLVS
FVQTLCKGLSQPTTNLVAGCLQLNPRTFLPEQNQDMPPHLPTASASFPVHPYSYQSPG
LPSPPYGTMDSSHVFHVKPPPHAYSAALEPFFESPLTDCTSPSFDGPLSPPLSINGNFSF
KHEPSAEFEKNYAFTMHYPAATLAGAQSHGSIFSGTAAPRCEIPIDNIMSFDSHSHHER
VMSAQLNAIFHD.

SEQ ID NO.2 (mNeuroD1 Amino Acid Sequence):

MTKSYSESGLMGEPQPQGPPSWTDECLSSQDEEHEADKKEDELEAMNAEEDSL
RNGGEEEEEDEDLEEEEEEEEEEDQKPKRRGPKKKKMTKARLERFKLRRMKANAR
ERNRMHGLNAALDNLRKVVPCYSKTQKLSKIETLRLAKNYIWALSEILRSGKSPDLVS
FVQTLCKGLSQPTTNLVAGCLQLNPRTFLPEQNPDMPPHLPTASASFPVHPYSYQSPGL
PSPPYGTMDSSHVFHVKPPPHAYSAALEPFFESPLTDCTSPSFDGPLSPPLSINGNFSFK
HEPSAEFEKNYAFTMHYPAATLAGPQSHGSIFSSGAAAPRCEIPIDNIMSFDSHSHHER
VMSAQLNAIFHD.

SEQ ID NO.3 (hNeuroD1 Nucleotide Sequence):

atgaccaaatcgtacagcgagagtgggctgatgggcgagcctcagccccaaggtcctccaagctggacagacgagtgtctca
gttctcaggacgaggagcacgaggcagacaagaaggaggacgacctcgaaaccatgaacgcagaggaggactcactgaggaacg
ggggagaggaggaggacgaagatgaggacctggaagaggaggaagaagaggaagaggaggatgacgatcaaaagcccaaga

gacgcggccccaaaaagaagaagatgactaaggctcgcctggagcgttttaaattgagacgcatgaaggctaacgcccgggagcgg

aaccgcatgcacggactgaacgcggcgctagacaacctgcgcaaggtggtgccttgctattctaagacgcagaagctgtccaaaatc

gagactctgcgcttggccaagaactacatctgggctctgtcggagatcctgcgctcaggcaaaagcccagacctggtctccttcgttca

gacgctttgcaagggcttatcccaacccaccaccaacctggttgcgggctgcctgcaactcaatcctcggactttctgcctgagcagaa

ccaggacatgcccccccacctgccgacggccagcgcttccttccctgtacacccctactcctaccagtcgcctgggctgcccagtccg

ccttacggtaccatggacagctcccatgtcttccacgttaagcctccgccgcacgcctacagcgcagcgctggagcccttctttgaaag

ccctctgactgattgcaccagcccttcctttgatggaccccctcagcccgccgctcagcatcaatggcaacttctctttcaaacacgaaccg

tccgccgagtttgagaaaaattatgcctttaccatgcactatcctgcagcgacactggcaggggcccaaagccacggatcaatcttctca

ggcaccgctgcccctcgctgcgagatccccatagacaatattatgtccttcgatagccattcacatcatgagcgagtcatgagtgcccag

ctcaatgccatatttcatgattag.

SEQ ID NO.4 (mNeuroD1 Nucleotide Sequence):

atgaccaaatcatacagcgagagcgggctgatgggcgagcctcagccccaaggtcccccaagctggacagatgagtgtctca

gttctcaggacgaggaacacgaggcagacaagaaagaggacgagcttgaagccatgaatgcagaggaggactctctgagaaacgg

gggagaggaggaggaggaagatgaggatctagaggaagaggaggaagaagaagaggaggaggaggatcaaaagcccaagag

acggggtcccaaaaagaaaaagatgaccaaggcgcgcctagaacgttttaaattaaggcgcatgaaggccaacgcccgcgagcgg

aaccgcatgcacgggctgaacgcggcgctggacaacctgcgcaaggtggtaccttgctactccaagacccagaaactgtctaaaata

gagacactgcgcttggccaagaactacatctgggctctgtcagagatcctgcgctcaggcaaaagcccctgatctggtctccttcgtaca

gacgctctgcaaaggtttgtcccagcccactaccaatttggtcgccggctgcctgcagctcaaccctcggactttcttgcctgagcagaa

cccggacatgccccccgcatctgccaaccgccagcgcttccttcccggtgcatccctactcctaccagtcccctggactgcccagcccg

ccctacggcaccatggacagctcccacgtcttccacgtcaagccgccgccacacgcctacagcgcagctctggagcccttctttgaaa

gcccccctaactgactgcaccagcccttcctttgacggacccctcagcccgccgctcagcatcaatggcaacttctctttcaaacacgaac

catccgccgagtttgaaaaaaattatgcctttaccatgcactaccctgcagcgacgctggcagggcccaaagccacggatcaatcttc

tcttccggtgccgctgcccctcgctgcgagatccccatagacaacattatgtctttcgatagccattcgcatcatgagcgagtcatgagtg

cccagcttaatgccatctttcacgattag.

SEQ ID NO.5 (hBrn2 Amino Acid Sequence):

MATAASNHYSLLTSSASIVHAEPPGGMQQGAGGYREAQSLVQGDYGALQSNGH
PLSHAHQWITALSHGGGGGGGGGGGGGGGGGGGGGGDGSPWSTSPLGQPDIKPSVVV
QQGGRGDELHGPGALQQQHQQQQQQQQQQQQQQQQQQQQQQRPPHLVHHAANHHP
GPGAWRSAAAAAHLPPSMGASNGGLLYSQPSFTVNGMLGAGGQPAGLHHHGLRDA
HDEPHHADHHPHPHSHPHQQPPPPPPPQGPPGHPGAHHDPHSDEDTPTSDDLEQFAK
QFKQRRIKLGFTQADVGLALGTLYGNVFSQTTICRFEALQLSFKNMCKLKPLLNKWL
EEADSSSGSPTSIDKIAAQGRKRKKRTSIEVSVKGALESHFLKCPKPSAQEITSLADSLQ
LEKEVVRVWFCNRRQKEKRMTPPGGTLPGAEDVYGGSRDTPPHHGVQTPVQ.

SEQ ID NO.6 (mBrn2 Amino Acid Sequence):

MATAASNHYSLLTSSASIVHAEPPGGMQQGAGGYREAQSLVQGDYGALQSNGH
PLSHAHQWITALSHGGGGGGGGGGGGGGGGGGGGGGDGSPWSTSPLGQPDIKPSVVV
QQGGRGDELHGPGALQQQHQQQQQQQQQQQQQQQQQQQQQQRPPHLVHHAAN
HHPGPGAWRSAAAAAHLPPSMGASNGGLLYSQPSFTVNGMLGAGGQPAGLHHHGL
RDAHDEPHHADHHPHPHSHPHQQPPPPPPPQGPPGHPGAHHDPHSDEDTPTSDDLEQ
FAKQFKQRRIKLGFTQADVGLALGTLYGNVFSQTTICRFEALQLSFKNMCKLKPLLNK
WLEEADSSSGSPTSIDKIAAQGRKRKKRTSIEVSVKGALESHFLKCPKPSAQEITSLAD
SLQLEKEVVRVWFCNRRQKEKRMTPPGGTLPGAEDVYGGSRDTPPHHGVQTPVQ.

SEQ ID NO.7 (hBrn2 Nucleotide Sequence):

atggcgaccgcagcgtctaaccactacagcctgctcacctccagcgcctccatcgtgcacgccgagccgcccggcggcatgc

agcagggcgcggggggggctaccgcgaagcgcagagcctggtgcagggcgactacggcgctctgcagagcaacggacacccgctc

agccacgctcaccagtggatcaccgcgctgtcccacggcggcggcggcggggggcggtggcggcggcggggggggcggggggc

ggcggcggggggcggcggcgacggctccccgtggtccaccagccccctgggccagccggacatcaagccctcggtggtggtgcag

cagggcggccgcggagacgagctgcacgggccaggcgccctgcagcagcagcatcagcagcagcaacagcaacagcagcagc

aacagcagcaacagcagcagcagcagcaacagcggccgccgcatctggtgcaccacgccgctaaccaccacccgggaccc

ggggcatggcggagcgcggcggctgcagcgcacctccaccctccatgggagcgtccaacggcggcttgctctactcgcagccca

gcttcacggtgaacggcatgctgggcgccggcgggcagccggccggtctgcaccaccacggcctgcgggacgcgcacgacgag

ccacaccatgccgaccaccacccgcacccgcactcgcacccacaccagcagccgccgcccccgccgcccccgcagggtccgcct

ggccacccaggcgcgcaccacgacccgcactcggacgaggacacgccgacctcggacgacctggagcagttcgccaagcagttc

aagcagcggcggatcaaactgggatttacccaagcggacgtggggctggctctgggcaccctgtatggcaacgtgttctcgcagacc

accatctgcaggtttgaggccctgcagctgagcttcaagaacatgtgcaagctgaagcctttgttgaacaagtggttggaggaggcgg

actcgtcctcgggcagccccacgagcatagacaagatcgcagcgcaagggcgcaagcggaaaaagcggacctccatcgaggtga

gcgtcaagggggctctggagagccatttcctcaaatgccccaagccctcggcccaggagatcacctccctcgcggacagcttacagc

tggagaaggaggtggtgagagtttggttttgtaacaggagacagaaagagaaaaggatgacccctcccggagggactctgccgggc

gccgaggatgtgtacggggggagtagggacactccaccacaccacggggtgcagacgcccgtccagtga.

SEQ ID NO.8 (mBrn2 Nucleotide Sequence):

atggcgaccgcagcgtctaaccactacagcctgctcacctccagcgcctccatcgtacatgccgagccgcctggcggcatgc
agcagggcgcaggggggctaccgcgaggcgcagagcctggtgcagggcgactacggcgcgctgcagagcaacgggcacccgct
cagccacgctcaccagtggatcaccgcgctgtcccacggcggcggcggcggggggcggcggcggcggtggaggaggcggggga
ggcggcgggggaggcggcgacggctccccgtggtccaccagccccctaggccagccggacatcaagccctcggtggtggtacag
cagggtggccgaggcgacgagctgcacgggccaggagcgctgcagcaacagcatcaacagcaacagcaacagcagcagcagca
gcagcagcagcagcagcaacagcagcagcaacaacagcgaccgccacatctggtgcaccacgctgccaaccaccatcccgg
gcccggggcatggcggagtgcggcggctgcagctcacctccctccctccatgggagcttccaacggcggtttgctctattcgcagcc
gagcttcacggtgaacggcatgctgggcgcaggagggcagccggctgggctgcaccaccacggcctgagggacgcccacgatga
gccacaccatgcagaccaccacccgcatccgcactctcacccacaccagcaaccgcccccgccacctcccccacaaggcccaccg
ggccacccaggcgcgcaccacgacccgcactcggacgaggacacgccgacctcagacgacctggagcagttcgccaagcaattc
aagcagaggcggatcaaactcggatttactcaagcagacgtggggctggcgcttggcaccctgtacggcaacgtgttctcgcagacc
accatctgcaggtttgaggccctgcagctgagcttcaagaacatgtgcaagctgaagcctttgttgaacaagtggttggaagaggcaga
ctcatcctcgggcagccccaccagcatagacaagatcgcagcgcaagggcgcaaacggaaaaagcggacctccatcgaggtgagc
gtcaaggggggctctggagagccatttcctcaaatgccctaagccctcggcccaggagatcacctccctcgcggacagcttacagctgg
agaaggaggtggtgagagtttggtttttgtaacaggagacagaaagagaaaaggatgacccctcccggagggactctgccgggcgcc
gaggatgtgtatggggggtagtagggacacgccaccacaccacgggggtgcagacgcccgtccagtga.

SEQ ID NO.9 (hAscl1 Amino Acid Sequence):

MESSAKMESGGAGQQPQPQPQQPFLPPAACFFATAAAAAAAAAAAAAAQSAQQQ
QQQQQQQQQAPQLRPAADGQPSGGGHKSAPKQVKRQRSSSPELMRCKRRLNFSGFG
YSLPQQQPAAVARRNERERNRVKLVNLGFATLREHVPNGAANKKMSKVETLRSAVEY
IRALQQLLDEHDAVSAAFQAGVLSPTISPNYSNDLNSMAGSPVSSYSSDEGSYDPLSPE
EQELLDFTNWF.

SEQ ID NO.10 (mAscl1 Amino Acid Sequence):

MESSGKMESGAGQQPQPPQPFLPPAACFFATAAAAAAAAAAAQSAQQQQPQA
PPQQAPQLSPVADSQPSGGGHKSAAKQVKRQRSSSPELMRCKRRLNFSGFGYSLPQQ
QPAAVARRNERERNRVKLVNLGFATLREHVPNGAANKKMSKVETLRSAVEYIRALQQ
LLDEHDAVSAAFQAGVLSPTISPNYSNDLNSMAGSPVSSYSSDEGSYDPLSPEEQELL
DFTNWF.

SEQ ID NO.11 (hAscl1 Nucleotide Sequence):

atggaaagctctgccaagatggagagcggcggcgccggccagcagccccagccgcagccccagcagcccttcctgccgcc

cgcagcctgtttctttgccacggccgcagccgcggcggccgcagccgccgcagcggcagcgcagagcgcgcagcagcagc

agcagcagcagcagcagcaggcgccgcagctgagaccggcggccgacggccagccctcaggggcggtcacaagtcagc

gcccaagcaagtcaagcgacagcgctcgtcttcgcccgaactgatgcgctgcaaacgccggctcaacttcagcggctttggctacag

cctgccgcagcagcagccggccgccgtggcgcgccgcaacgagcgcgagcgcaaccgcgtcaagttggtcaacctgggctttgcc

acccttcgggagcacgtccccaacggcgcgggccaacaagaagatgagtaaggtggagacactgcgctcggcggtcgagtacatcc

gcgcgctgcagcagctgctggacgagcatgacgcggtgagcgccgccttccaggcaggcgtcctgtcgcccaccatctcccccaac

tactccaacgacttgaactccatggccggctcgccggtctcatcctactcgtcggacgagggctcttacgacccgctcagccccgagg

agcaggagcttctcgacttcaccaactggttctga.

SEQ ID NO.12 (mAscl1 Nucleotide Sequence):

atggagagctctggcaagatggagagtggagccggccagcagccgcagcccccgcagcccttcctgcctcccgcagcctgc

ttctttgcgaccgcggcggcggcggcagcggcggcggccgcggcagctcagagcgcgcagcagcaacagccgcaggcgccgcc

gcagcaggcgccgcagctgagcccggtggccgacagccagccctcaggggcggtcacaagtcagcggccaagcaggtcaagc

gccagcgctcgtcctctccggaactgatgcgctgcaaacgccggctcaacttcagcggcttcggctacagcctgccacagcagcagc

cggccgccgtggcgcgccgcaacgagcgcgagcgcaaccgggtcaagttggtcaacctgggttttgccaccctccgggagcatgtc

cccaacggcgcgggccaacaagaagatgagcaaggtggagacgctgcgctcggcggtcgagtacatccgcgcgctgcagcagctg

ctggacgagcacgacgcggtgagcgctgcctttcaggcgggcgtcctgtcgcccaccatctcccccaactactccaacgacttgaact

ctatggcggggttctccggtctcgtcctactcctccgacgagggatcctacgaccctcttagcccagaggaacaagagctgctggacttta

ccaactggttctga.

SEQ ID NO.13 (hNgn2 Amino Acid Sequence):

MFVKSETLELKEEEDVLVLLGSASPALAALTPLSSSADEEEEEEPGASGGARRQR

GAEAGQGARGGVAAGAEGCRPARLLGLVHDCKRRPSRARAVSRGAKTAETVQRIKK

TRRLKANNRERNRMHNLNAALDALREVLPTFPEDAKLTKIETLRFAHNYIWALTETLR

LADHCGGGGGGLPGALFSEAVLLSPGGASAALSSSGDSPSPASTWSCTNSPAPSSSVSS

NSTSPYSCTLSPASPAGSDMDYWQPPPPDKHRYAPHLPIARDCI.

SEQ ID NO.14 (mNgn2 Amino Acid Sequence):

MFVKSETLELKEEEEVLMLLGSASPASATLTPMSSSADEEEDEELRRPGSARGQR GAEAGQGVQGSPASGAGGCRPGRLLGLMHECKRRPSRSRAVSRGAKTAETVQRIKKT RRLKANNRERNRMHNLNAALDALREVLPTFPEDAKLTKIETLRFAHNYIWALTETLRL ADHCAGAGGLQGALFTEAVLLSPGAALGASGDSPSPPSSWSCTNSPASSSNSTSPYSC TLSPASPGSDVDYWQPPPPEKHRYAPHLPLARDCI.

SEQ ID NO.15 (hNgn2 Nucleotide Sequence):

atgttcgtcaaatccgagaccttggagttgaaggaggaagaggacgtgttagtgctgctcggatcggcctcccccgccttggcg gccctgaccccgctgtcatccagcgccgacgaagaagaggaggaggagccgggcgcgtcaggcggggcgcgtcggcagcgcg gggctgaggccgggcaggggggcgcggggcggcgtggctgcgggtgcggagggctgccggcccgcacggctgctgggtctggta cacgattgcaaacggcgcccttcccgggcgcgggccgtctcccgaggcgccaagacggccgagacggtgcagcgcatcaagaag acccgtagactgaaggccaacaaccgcgagcgaaaccgcatgcacaacctcaacgcggcactggacgcgctgcgcgaggtgctcc ccacgttccccgaggacgccaagctcaccaagatcgagaccctgcgcttcgcccacaactacatctgggcactcaccgagaccctgc gcctggcggatcactgcgggggcggcggcggggggcctgccggggggcgctcttctccgaggcagtgttgctgagcccgggaggag ccagcgccgccctgagcagcagcggagacagcccctcgcccgcctccacgtggagttgcaccaacagccccgcgccgtcctcctc cgtgtcctccaattccacctcccccctacagctgcactttatcgcccgccagcccggccgggtcagacatggactattggcagcccccac ctcccgacaagcaccgctatgcacctcacctccccatagccagggattgtatctag.

SEQ ID NO.16 (mNgn2 Nucleotide Sequence):

atgttcgtcaaatctgagactctggagttgaaggaggaagaggaggtactgatgctgctgggctcggcttccccggcctcggc gaccctgaccccgatgtcctccagcgcgggacgaggaggaggacgaggagctgcgccggccgggctccgcgcgtgggcagcgtg gagcggaagccgggcaggggggtgcagggcagtccggcgtcgggtgccggggggttgccggccagggcggctgctgggcctgatg cacgagtgcaagcgtcgcccgtcgcgctcacgggccgtctcccgaggtgccaagacggcggagacggtgcagcgcatcaagaag acccgcaggctcaaggccaacaaccgcgagcgcaaccgcatgcacaacctaaacgccgcgctggacgcgctgcgcgaggtgctg cccaccttccccgaggatgccaagctcacgaagatcgagacgctgcgcttcgcccacaattacatctgggcgctcaccgagactctgc gcctggcggaccactgcgccggcgccggtggcctccaggggggcgctcttcacggaggcggtgctcctgagcccgggagctgcgct cggcgccagcggggacagcccttctccaccttcctcctggagctgcaccaacagcccggcgtcatcctccaactccacgtccccatac agctgcactttatcgcccgctagccccgggtcagacgtggactactggcagcccccacctccggagaagcatcgttatgcgcctcacc tgccctcgccagggactgtatctag.

SEQ ID NO.17:

taatcccacctccctctctgtgctgggactcacagagggagacctcaggaggcagtctgtccatcacatgtccaaatgcagagc

atacctgggctgggcgcagtggcgcacaactgtaattccagcactttgggaggctgatgtggaaggatcacttgagcccagaagttct

agaccagcctgggcaacatggcaagaccctatctctacaaaaaaagttaaaaaatcagccacgtgtggtgacacacacctgtagtccc

agctattcaggaggctgaggtgaggggatcacttaaggctgggaggttgaggctgcagtgagtcgtggttgcgccactgcactccag

cctgggcaacagtgagaccctgtctcaaaagacaaaaaaaaaaaaaaaaaaaaagaacatatcctggtgtggagtaggggacgct

gctctgacagaggctcggggggcctgagctggctctgtgagctggggaggaggcagacagccaggccttgtctgcaagcagacctg

gcagcattgggctggccgcccccccagggcctcctcttcatgcccagtgaatgactcaccttggcacagacacaatgttcggggtgggc

acagtgcctgcttcccgccgcaccccagcccccctcaaatgccttccgagaagcccattgagcaggggggcttgcattgcaccccagc

ctgacagcctggcatcttgggataaaagcagcacagcccccctaggggctgcccttgctgtgtggcgccaccggcggtggagaacaa

ggctctattcagcctgtgcccaggaaaggggatcaggggatgcccaggcatggacagtgggtggcagggggggagaggagggct

gtctgcttcccagaagtccaaggacacaaatgggtgaggggactgggcagggttctgaccctgtgggaccagagtggagggcgtag

atggacctgaagtctccagggacaacagggcccaggtctcaggctcctagttgggcccagtggctccagcgtttccaaacccatccat

ccccagaggttcttcccatctctccaggctgatgtgtgggaactcgaggaaataaatctccagtgggagacggagggggtggccaggg

aaacggggcgctgcaggaataaagacgagccagcacagccagctcatgtgtaacggctttgtggagctgtcaaggcctggtctctgg

gagagaggcacagggaggccagacaaggaaggggtgacctggagggacagatccaggggctaaagtcctgataaggcaagaga

gtgccggccccctcttgccctatcaggacctccactgccacatagaggccatgattgacccttagacaaagggctggtgtccaatccca

gcccccagccccagaactccagggaatgaatgggcagagagcaggaatgtgggacatctgtgttcaagggaaggactccaggagt

ctgctgggaatgaggcctagtaggaaatgaggtggcccttgagggtacagaacaggttcattcttcgccaaattcccagcaccttgcag

gcacttacagctgagtgagataatgcctgggttatgaaatcaaaaagttggaaagcaggtcagaggtcatctggtacagcccttccttcc

ctttttttttttttttttttgtgagacaaggtctctctctgttgcccaggctggagtggcgcaaacacagctcactgcagcctcaacctactggg

ctcaagcaatcctccagcctcagcctcccaaagtgctgggattacaagcatgagccaccccactcagccctttccttcctttttaattgatg

cataataattgtaagtattcatcatggtccaaccaacccttcttgacccaccttcctagagagaggtcctcttgcttcagcggtcagggc

cccagacccatggtctggctccaggtaccacctgcctcatgcaggagttggcgtgcccaggaagctctgcctctgggcacagtgacct

cagtggggtgaggggagctctccccatagctgggctgcggcccaacccacccccctcaggctatgccaggggtgttgccagggg

cacccgggcatcgccagtctagcccactccttcataaagccctcgcatcccaggagcgagcagagccagagcaggttggagaggag

acgcatcacctccgctgctcgcgg.

SEQ ID NO.18 (h-SA-Ascl1 Amino Acid Sequence):

MESSAKMESGGAGQQPQPQPQQPFLPPAACFFATAAAAAAAAAAAAAAQSAQQQ

QQQQQQQQQQAPQLRPAADGQPSGGGHKSAPKQVKRQRSSAPELMRCKRRLNFSGFG

YSLPQQQPAAVARRNERERNRVKLVNLGFATLREHVPNGAANKKMSKVETLRSAVEY

IRALQQLLDEHDAVSAAFQAGVLAPTIAPNYSNDLNSMAGAPVSSYSSDEGSYDPLAP

EEQELLDFTNWF.

SEQ ID NO.19 (h-SA-Ascl1 Nucleotide Sequence):

atggaaagctctgccaagatggagagcggcggcgccggccagcagccccagccgcagccccagcagcccttcctgccgcc

cgcagcctgtttctttgccacggccgcagccgcggcggccgcagccgccgcagcggcagcgcagagcgcgcagcagcagcagc

agcagcagcagcagcagcaggcgccgcagctgagaccggcggccgacggccagccctcaggggggcggtcacaagtcagc

gcccaagcaagtcaagcgacagcgctcgtctgcacccgaactgatgcgctgcaaacgccggctcaacttcagcggctttggctacag

cctgccgcagcagcagccggccgccgtggcgcgccgcaacgagcgcgagcgcaaccgcgtcaagttggtcaacctgggctttgcc

acccttcgggagcacgtccccaacggcgcggccaacaagaagatgagtaaggtggagacactgcgctcggcggtcgagtacatcc

gcgcgctgcagcagctgctggacgagcatgacgcggtgagcgccgccttccaggcaggcgtcctggcacccaccatcgcacccaa

ctactccaacgacttgaactccatggccggcgcaccggtctcatcctactcgtcggacgagggctcttacgacccgctcgcacccgag

gagcaggagcttctcgacttcaccaactggttctga.

**Cell Culture**

For the human glioma cell lines U251 and U87 (purchased from the Cell Bank of Shanghai Institute of Life Sciences, Chinese Academy of Sciences) and normal human astrocytes (HA cells) (purchased from Sciencell), the cells were cultured at 37° C in a 5% CO2 incubator. The culture medium used was DMEM supplemented with 10% fetal bovine serum and 1% penicillin/streptomycin. After viral infection, the medium was completely replaced with induction medium (DMEM, 2% B-27, 1% PS) after 12 hours, and after 48 hours, it was again replaced with neural culture medium (DMEM/F-12, 2% B-27, 1% PS, 20 ng/mL BDNF, 20 ng/mL GDNF). Thereafter, half of the culture medium was changed every three days.

**Immunostaining**

Immunostaining of cultured cells was conducted following the methods referenced in "Direct conversion of fibroblasts to functional neurons by defined factors" (Vierbuchen, T. et al. Nature 463, 1035-1041 (2010)). The immunostaining of tissue sections was performed according to previously published methods. The primary antibodies used for immunostaining included: mouse anti-NeuN (Millipore, 1:100), rabbit anti-Dsred (Clontech, 1:500), mouse anti-Tuj1 (Covance, 1:500), mouse anti-Map2 (Sigma, 1:500), rabbit anti-GFP (Invitrogen, 1:1,000), chick anti-GFP (Invitrogen, 1:1,000), rabbit anti-Ki67 (1:200; RM-9106; Thermo Fisher Scientific), mouse anti-BrdU (1:200; B2531; Sigma). FITC-, Cy3-, and Cy5-conjugated secondary antibodies were purchased from Jackson Immunoresearch.

**MTT Assay Method**

On the first day, the cell density was adjusted to $5 \times 10^4$ cells/mL and 100 μL was plated per well in a 96-well plate. On the second day, the culture medium was removed, and $5 \times 10^3$ cells/well were treated with the corresponding viruses diluted in a tenfold MOI (multiplicity of infection) gradient, with three replicates for each MOI. On the seventh day, the supernatant was gently removed, and 50 μL of culture medium was added to each well, followed by 20 μL of 5 mg/mL MTT solution. After 3 hours, 100 μL of dissolving solution was added, and the plates were incubated overnight at 37° C to dissolve the formed formazan crystals, followed by measuring the OD value at 570 nm.

**Glioma Model**

For the subcutaneous glioma model, human U87 glioma cells were cultured, and during the logarithmic growth phase, they were inoculated into the axillae of nude mice. After two passages, the tumor tissue was collected under sterile conditions, and the tumor mass was cut into uniform small pieces approximately the size of grains of rice, which were then inoculated subcutaneously in the axillae of nude mice using an implantation needle. Once the tumor grew to approximately 100 mm$^3$, the mice were randomly grouped, and the treatment began. All samples were dissolved in PBS, with an injection volume of 50 µL/tumor. The length and width of the tumor were measured every three days, and the tumor volume was calculated using the formula:

$$Volume = (length*width\textasciicircum2)/2$$

The tumor inhibition rate was calculated using the following formula:

$$Tumor\ Inhibition\ Rate\% = (V_{model\ group} - V_{treatment\ group})/\ V_{model\ group} *100\%$$

Animals were subsequently euthanized, and tumor masses were weighed for biochemical and molecular detection.

For the orthotopic glioma model, 7-week-old NOD-scid mice were used. Human glioma cells, either induced with 0.25% trypsin for 3 days or not induced, were centrifuged to remove the supernatant, resulting in a cell density of approximately 2.5 × 10^5 cells/µL. A total of 2 µL (5 × 10^5 cells) was implanted into the striatum of each mouse. After three weeks, histological analysis or viral injection was performed, followed by immunohistochemical detection.

**Example 1: Construction and Packaging of Recombinant Oncolytic Virus (Containing Recombinant Nucleic Acid)**

This example involves recombinant oncolytic viruses, using adenovirus type 5 (Ad5) as an example for verification. In the recombinant oncolytic adenovirus genome, the human GFAP promoter sequence (nucleotide sequence shown as SEQ ID NO.17) replaces the endogenous promoter of the wild-type E1A gene, with the fragments derived from human Ascll (SEQ ID No.11) and the CDS of the human Ngn2 gene (SEQ ID No.15) constructed into the vector. P2A is a self-cleaving peptide that enables efficient co-expression of hAscl1 and Ngn2, resulting in Ad5-AN, while the empty vector Ad5-Vector serves as a control.

Adenovirus packaging and purification were performed by co-transfecting the adenovirus packaging plasmid and shuttle plasmid into HEK-293 cells. When the cells formed plaques, the cell supernatant and lysate were collected and purified via concentration or cesium chloride density gradient centrifugation. The adenovirus titer was determined using an enzyme-linked immunosorbent assay (ELISA), calculating the viral titer based on the number of positive cells that turned brown after infection.

**Example 2: Selective Killing Effect of Recombinant Oncolytic Virus on Cancer Cells**

In this example, human glioblastoma cells were selected as the tumor cell model. U251 and U87 glioma cell lines, along with normal human astrocytes (HA), were seeded in T25 tissue culture flasks, using a complete culture medium containing 10% fetal bovine serum and 1% penicillin/streptomycin. On the first day, the cell density was adjusted to 5 × 10^4 cells/mL, and 100 µL was plated per well in a 96-well plate. On the second day, the culture medium was removed, and the corresponding viruses (Ad5-vector, Ad5-AN) were diluted in a tenfold MOI gradient, added to each well for infection, with three replicates for each MOI. On the seventh day, the supernatant was gently removed, and 50 µL of culture medium was added to each well, followed by 20 µL of 5 mg/mL MTT solution. After 3 hours, 100 µL of dissolving solution was added, and the plates were incubated overnight at 37° C to dissolve the formed formazan crystals, followed by measuring the OD value at 570 nm.

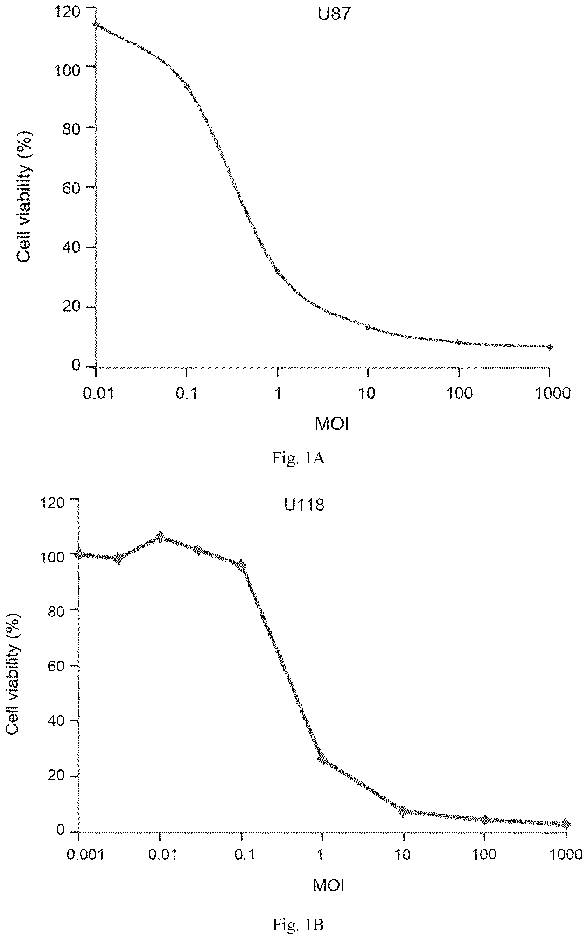

Results are shown in Figures 1A and 1B, indicating the killing curves of different infection multiplicities of Ad5-AN on U87 and U118 cells. The IC50 values of Ad5-AN on U87 and U118 cells were 0.9 and 0.6, respectively, demonstrating good tumor-killing ability, while the IC50 for normal human astrocytes HA was 136, indicating that Ad5-AN has good specificity and safety, allowing for a broader dosage range for subsequent treatments. The IC50 values of Ad5-vector on U87 and U118 cells were 0.8 and 0.7, respectively, and 116 for normal human astrocytes HA, also showing specific tumor-killing ability.

**Example 3: Reprogramming Effect of Recombinant Oncolytic Virus on Cancer Cells**

This example explores the application scheme of recombinant oncolytic viruses carrying transcription factors or combinations of transcription factors with high transduction efficiency to promote the trans-differentiation of glioma cells into non-tumorigenic cells, using the Ad5-AN oncolytic adenovirus with the combination of factors Ascll and Ngn2 as an example.

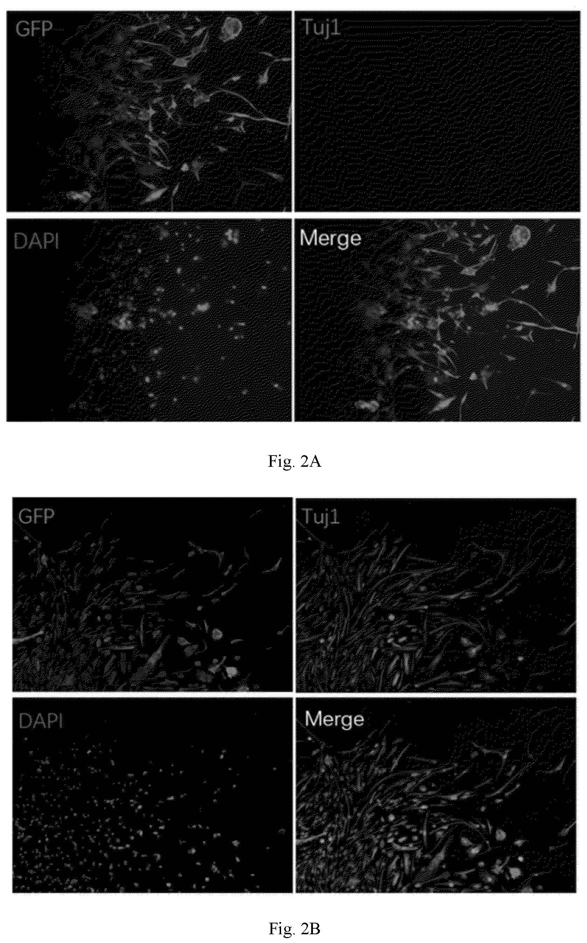

After 24 hours of culturing human glioma cells, adenovirus was added to observe its effect on the trans-differentiation of glioma cells into non-tumorigenic cells. A low-titer infection model (MOI of 0.05) was employed, and to better indicate the infected cells, a lentivirus carrying green fluorescent protein (FUGW-IRES-EGFP) was co-infected with the tumor cells. After 24 hours of infection, the culture medium was changed to DMEM/F12, B27, Glutamax, and penicillin/streptomycin. Brain-derived neurotrophic factor (BDNF; PeproTech, 20 ng/mL) was added to the medium every three days. After 10 days of viral infection, immunofluorescence detection of the cultured human glioma cells U251 revealed the presence of some Tuj1-positive cells (Tuj 1 is a neuronal marker), exhibiting neuronal morphology, as shown in Figures 2A and 2B. The results indicate that Ad5-AN can transdifferentiate glioma cells U251 into non-tumorigenic neuronal cells, with a trans-differentiation efficiency of 75.2%. In glioma cells infected with Ad5-vector, no Tuj1-positive cells were detected in the immunofluorescence analysis, indicating that the promotion of trans-differentiation of glioma cells into non-tumorigenic neuronal cells is specifically mediated by the combination of Ascll and Ngn2 factors.

**Example 4: Expression of reprogramming factor-expressing oncolytic adenovirus type 5 inhibits the growth of glioma tumor cells in a mouse ectopic implantation model.**

To verify the synergistic effect of oncolytic viruses and redifferentiation therapy, oncolytic viruses (using type 5 oncolytic adenovirus as an example) were used as vectors to express reprogramming factors. This approach takes advantage of the specific amplification of type 5 oncolytic adenoviruses in tumor cells, achieving a synergistic effect in suppressing glioma through oncolytic action and in vivo trans-differentiation therapy.

In this example, a human brain glioma U87 BALB/CA-nu mouse xenograft model was used. Cultured U87 human brain glioma cells were inoculated into the armpit of nude mice during the logarithmic growth phase. When the tumor reached approximately 100 $mm^3$ , appropriate tumor-bearing nude mice were randomly grouped. After grouping, treatment began, with the control group receiving PBS, the Ad5-AN-low group receiving a dose of $3\times10^8$ PFU, and the Ad5-vector-high and Ad5-AN-high groups receiving a dose of $1\times10^9$ PFU. Treatment was administered every two days for a total of five doses. Tumor volumes were measured and calculated every three days, and animals were euthanized later to weigh the tumor masses and perform biochemical and molecular tests.

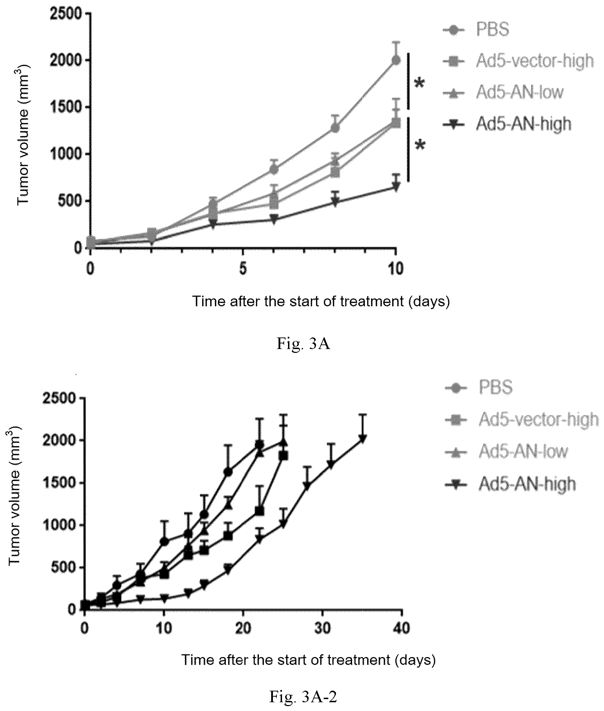

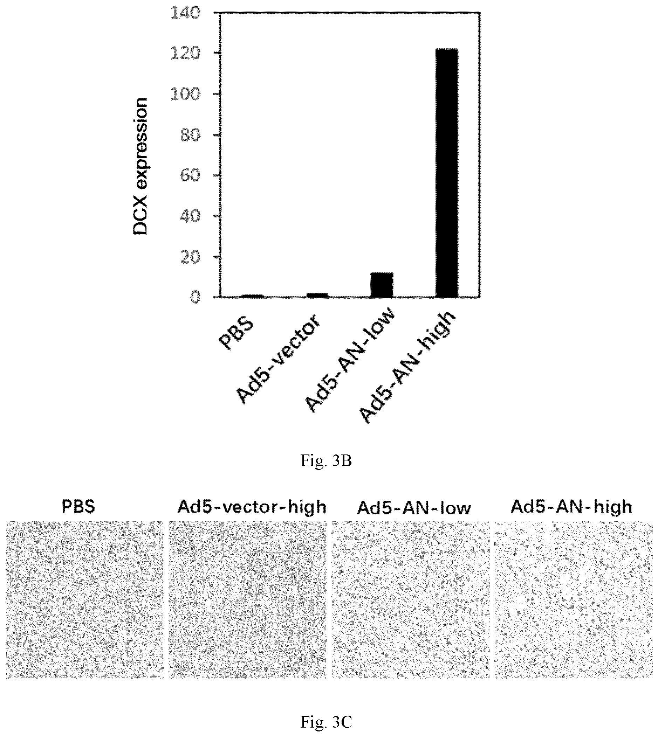

Results are shown in Figures 3A, 3B, and 3C. Figures 3A, 3B, and 3C demonstrate that the type 5 oncolytic adenovirus vector expressing reprogramming factors inhibited the growth of tumor cells in the glioma mouse xenograft model. It was found that compared to the control PBS group, the tumor volume in the Ad5-vector-high group decreased by 33.37%, the Ad5-AN-low group decreased by 32.25%, and the Ad5-AN-high group decreased by 67.49% (Figure 3A). The addition of reprogramming factors significantly enhanced the inhibitory effect of the oncolytic adenovirus on gliomas, leading to a substantial reduction in tumor cell growth. Realtime-PCR analysis also revealed that the early neuronal marker DCX was significantly elevated in the Ad5-AN-high group (Figure 3B), and HE staining showed that glioma growth was inhibited (Figure 3C). When the tumor in mice reached nearly 2000 $mm^3$ , animals were euthanized. The average time for the control PBS group was 21.3 days, the Ad5-empty vector group was 24.5 days, the Ad5-AN-low group was 23.6 days, and the Ad5-AN-high group was 35.4 days (Figure 3A-2). These results indicate that the synergistic action of oncolytic effects and in vivo reprogramming therapy achieved a more significant suppression of glioma growth.

Furthermore, for the human Ascll protein, if the five conserved serine-proline (SP) phosphorylation sites in its protein sequence (located at positions 93, 190, 194, 207, and 223 in the protein sequence) are mutated to alanine-proline (AP) (enhanced version, protein sequence SEQ ID NO: 18, nucleotide sequence SEQ ID NO: 19), the inhibitory effect of Ad5-AN on gliomas can be further improved.

**Example 5: Oncolytic virus combined drug therapy experiments expressing reprogramming factors.**

To explore the combined therapeutic effects of oncolytic viruses expressing reprogramming factors with existing treatments, this implementation takes human glioblastoma cells as an example. The oncolytic virus (specifically, adenovirus type 5) serves as a vector to express reprogramming factors, leveraging the specific amplification of adenovirus type 5 in tumor cells in combination with temozolomide.

In this example, a subcutaneous implantation model using human glioma U87 cells in BALB/c nu/nu mice was adopted. U87 human glioblastoma cells were inoculated into the armpits of the nude mice during the logarithmic growth phase. When the tumor grew to approximately 100 $mm^3$ , appropriate tumor-bearing mice were randomly grouped. After grouping, treatment commenced, with the PBS group serving as the control. The Ad5-AN group received a dose of $1 \times$

10^9 PFU, while the temozolomide (TMZ) gavage group received 15 mg/kg once daily for five days, followed by two days off. Additionally, there was a combination treatment group of Ad5-AN and temozolomide (Ad5-AN+TMZ), with Ad5-AN administered every two days for a total of five doses. Tumor volume was measured every three days, and later, the animals were euthanized to weigh the tumors and perform biochemical and molecular analyses.

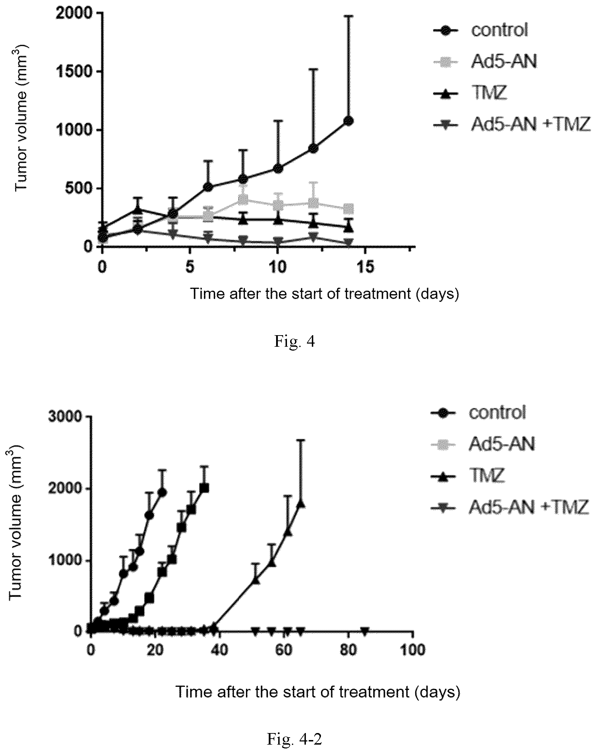

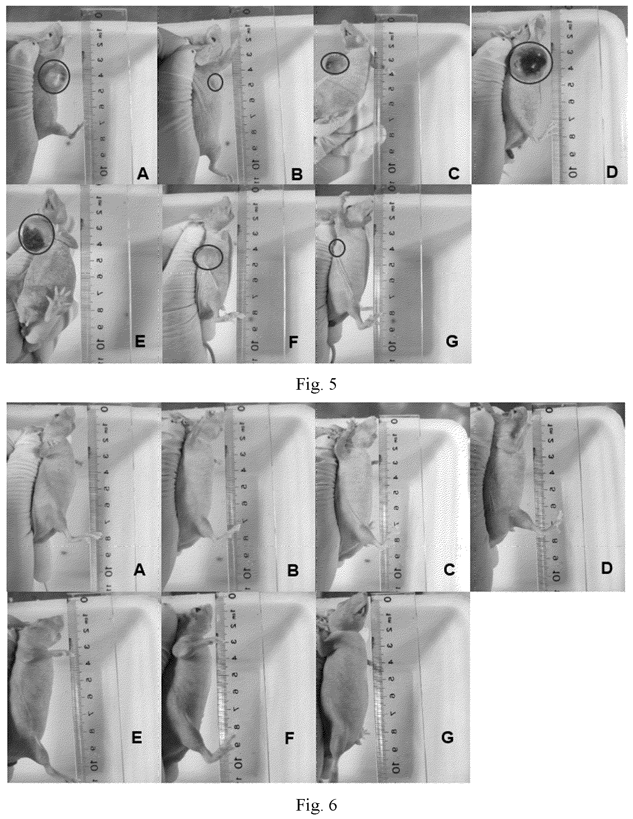

The results are shown in Figure 4, which illustrates the combined drug experiment using the oncolytic virus expressing reprogramming factors. It was found that compared to the control PBS group, the tumor volume in the Ad5-AN group significantly decreased, and the temozolomide group also exhibited a significant reduction in tumor volume. Moreover, the combination treatment group (Ad5-AN and temozolomide) showed a markedly better effect than either the Ad5-AN group or the temozolomide group alone, with tumors nearly disappearing (4 out of 6 cases). Furthermore, after extending the experiment (Figure 4-2), it was observed that tumors in the temozolomide group gradually relapsed around 35 days post-treatment, particularly after 56 days when tumors recurred in all mice from the seven parallel experimental groups (Figure 5, 7/7). By around 60 days, tumor growth approached 2000 mm$^3$ , which aligns with the high recurrence rates of gliomas following temozolomide chemotherapy in clinical settings. In contrast, the tumor volume in the combination treatment group (Ad5-AN and temozolomide) remained significantly lower compared to the Ad5-AN and temozolomide monotherapy groups, with tumors essentially disappearing (7/7 cases). Importantly, after 93 days, none of the tumors in the seven parallel experimental groups had recurred (Figure 6, 0/7). These results indicate that the oncolytic virus expressing reprogramming factors can be combined with existing therapies to achieve a better antitumor effect.

**Example 6: Tumor orthotopic model of oncolytic virus expressing reprogramming factors.**

To further confirm the therapeutic potential of oncolytic viruses expressing reprogramming factors against tumors, this implementation uses a human glioblastoma cell brain orthotopic model. The oncolytic virus (specifically, adenovirus type 5) serves as a vector to express reprogramming factors, leveraging the specific amplification of adenovirus type 5 in tumor cells to validate the synergistic effect of oncolytic action and in vivo trans-differentiation therapy in inhibiting gliomas.

In this example, glioma cells (U87-luc) were first implanted into the brain (5 × 10^5 cells). After 7 days of implantation, the mice were grouped for treatment, with the PBS group serving as the control. The Ad5-AN group received a dose of 1 × 10^9 PFU, while the temozolomide (TMZ) gavage group received 15 mg/kg once daily for five days, followed by two days off. Additionally, there was a combination treatment group of Ad5-AN and temozolomide, with Ad5-AN administered every four days for a total of three doses. 30 days post-virus injection, immunohistochemical analysis of brain tissue from the Ad5-AN group revealed that infected cells expressed the neuronal marker Tuj1, displaying neuronal morphology. Meanwhile, the survival of mice injected with Ad5-AN was significantly longer than that of the PBS group (average 32.6 days vs. 22.3 days). Furthermore, the combination treatment group of Ad5-AN and temozolomide significantly extended survival compared to the temozolomide monotherapy group (average 97.5 days vs. 56.8 days). These results indicate that even in the context of orthotopic tumors, the oncolytic virus expressing reprogramming factors can be effectively combined with existing therapies, achieving better antitumor effects and long-term resistance to recurrence.

**Example 7: Oncolytic Virus Vector Expressing Reprogramming Factors Inhibits Tumor Cell Growth in Glioma PDX Models**

To further validate the combined effect of oncolytic viruses and redifferentiation therapy, this implementation was tested in a patient-derived tumor xenograft (PDX) model constructed from human glioma tissue. PDX tissues were inoculated into the armpits of nude mice for passaging, and when they reached the logarithmic growth phase, they were re-inoculated into the armpits of nude mice. When the tumor grew to approximately 100 mm$^3$ , appropriate tumor-bearing mice were randomly grouped. After grouping, treatment commenced, with the PBS group serving as the control. The Ad5-AN group received a dose of 1 × 10^9 PFU, while the temozolomide (TMZ) gavage group received 15 mg/kg once daily for five days, followed by two days off. Additionally, there was a combination treatment group of Ad5-AN and temozolomide, with Ad5-AN administered every two days for a total of five doses. Tumor volume was measured every three days, and later, the animals were euthanized to weigh the tumors and perform biochemical and molecular analyses. The results showed that, similar to the subcutaneous models with glioma cell lines, the tumor volume in the Ad5-AN group was significantly reduced compared to the control PBS group. The tumor volume in the temozolomide group also decreased significantly; however, around 45 days post-treatment, tumors gradually relapsed and began to regrow (6/6 cases), reaching nearly 2000 mm$^3$ by around 72 days. In contrast, the combination treatment group of Ad5-AN and temozolomide demonstrated a significantly lower tumor volume compared to both the Ad5-AN and temozolomide monotherapy groups, with tumors essentially disappearing (72 days, 5/6 cases). More importantly, the observation indicated a sustained antitumor effect (102 days, 4/6 cases with no recurrence). These results suggest that the oncolytic virus expressing reprogramming factors can be effectively combined with existing therapies to achieve better antitumor effects and long-term resistance to recurrence in in vivo models derived from patient tissues.

**Example 8: Recombinant Herpes Simplex Virus Expressing Reprogramming Factors Inhibits Glioma Growth**

In this implementation, type 1 herpes simplex virus (HSV) was used as an oncolytic virus vector, with fragments from human Ascll (SEQ ID No. 11) and the coding sequence (CDS) of human Ngn2 (SEQ ID No. 15) inserted to achieve efficient co-expression of Ascll and Ngn2. After packaging and purifying the recombinant HSV, tests were conducted using a human glioblastoma U87 BALB/CA-nu mouse ectopic implantation model. When the tumor grew to approximately 100 mm$^3$, suitable tumor-bearing mice were randomly grouped for treatment. The groups included a control PBS group, an HSV (empty vector without reprogramming factors) group, and an HSV-AN group, all receiving a dose of $2 \times 10^6$ PFU. Additionally, there was a temozolomide (TMZ) gavage group (15 mg/kg once daily for five days with two days off), as well as a combination treatment group of HSV-AN and temozolomide. HSV was administered every two days for a total of five doses. Tumor volume was measured every three days, and later, the animals were euthanized to weigh the tumors and perform biochemical and molecular analyses. The results indicated that after 10 days of treatment, the tumor volume in the HSV empty vector group decreased by 25.6%, while the tumor volume in the HSV-AN group decreased by 58.9%. The addition of reprogramming factors significantly enhanced the inhibitory effect of the oncolytic herpes virus on gliomas. The temozolomide group showed a 73.6% reduction in tumor volume, but recurrence occurred after two months (6/6 cases). In contrast, the combination treatment group of HSV-AN and temozolomide exhibited a 92.5% reduction in tumor volume, with recurrence in only one case (1/6). These results demonstrate that the oncolytic action of herpes simplex virus, combined with the trans-differentiation of tumor cells in vivo, achieves a more pronounced ability to inhibit glioma growth. When combined with the existing therapy of TMZ, this approach provides better antitumor effects and long-term resistance to recurrence.

The above description outlines specific implementations of this invention, but it is not limited to these examples. Those skilled in the art may make various equivalent modifications or substitutions without departing from the spirit of the invention. Such equivalent modifications or substitutions are included within the scope defined by the claims of this application.

**Claims**

1. A recombinant oncolytic virus, wherein the recombinant oncolytic virus comprises recombinant nucleic acid, the recombinant nucleic acid comprising a functional fragment that promotes reprogramming/trans-differentiation of tumor cells into non-tumorigenic cells;
   The functional fragment comprises at least one functional fragment that promotes the expression of a transcription factor, wherein the functional fragment is selected from a functional fragment that promotes the expression of at least one transcription factor from NeuroD1, Brn2, Ascll, or Ngn2.

2. The recombinant oncolytic virus according to claim 1, wherein the recombinant nucleic acid comprises a set of functional fragments that synergistically promote reprogramming/trans-differentiation of tumor cells into non-tumorigenic cells;
   The functional fragment comprises at least two functional fragments that promote the expression of transcription factors, wherein the functional fragments are selected from functional fragments that promote the expression of at least two transcription factors from NeuroD1, Brn2, Ascll, or Ngn2.

3. The recombinant oncolytic virus according to claim 1, wherein the recombinant nucleic acid comprises a set of functional fragments that synergistically promote reprogramming/trans-differentiation of tumor cells into non-tumorigenic cells;
   The functional fragment comprises a functional fragment that promotes the expression of one or both transcription factors of Ascll and Ngn2.

4. The recombinant oncolytic virus according to claim 1, wherein the functional fragment is a polynucleotide encoding a functional protein, and the functional fragment is selected from a polynucleotide encoding a transcription factor with a sequence identity of not less than 75% to the sequence of SEQ ID NO.3, SEQ ID NO.4, SEQ ID NO.7, SEQ ID NO.8, SEQ ID NO.11, SEQ ID NO.12, SEQ ID NO.15, or SEQ ID NO.16;

   Preferably, the functional protein is selected from transcription factor functional proteins with a sequence identity of not less than 85% to SEQ ID NO.1, SEQ ID NO.2, SEQ ID NO.5, SEQ ID NO.6, SEQ ID NO.9, SEQ ID NO.10, SEQ ID NO.13, or SEQ ID NO.14;

More preferably, the functional fragment is a polynucleotide encoding a functional protein, wherein the functional fragment is selected from a polynucleotide encoding a transcription factor with a sequence identity of not less than 85% to SEQ ID NO.3, SEQ ID NO.4, SEQ ID NO.7, SEQ ID NO.8, SEQ ID NO.11, SEQ ID NO.12, SEQ ID NO.15, or SEQ ID NO.16;

More preferably, the functional protein is selected from transcription factor functional proteins with a sequence identity of not less than 95% to SEQ ID NO.1, SEQ ID NO.2, SEQ ID NO.5, SEQ ID NO.6, SEQ ID NO.9, SEQ ID NO.10, SEQ ID NO.13, or SEQ ID NO.14.

5. The recombinant oncolytic virus according to claim 4, wherein the functional fragment is a polynucleotide encoding a functional protein, and the functional fragment is selected from a polynucleotide encoding a transcription factor with a sequence identity of not less than 95% to SEQ ID NO.3, SEQ ID NO.4, SEQ ID NO.7, SEQ ID NO.8, SEQ ID NO.11, SEQ ID NO.12, SEQ ID NO.15, or SEQ ID NO. 16;

Preferably, the functional protein is selected from transcription factor functional proteins with a sequence identity of not less than 99% to SEQ ID NO.1, SEQ ID NO.2, SEQ ID NO.5, SEQ ID NO.6, SEQ ID NO.9, SEQ ID NO.10, SEQ ID NO.13, or SEQ ID NO.14.

6. The recombinant oncolytic virus according to claim 1, wherein the functional fragment is a polynucleotide encoding a functional protein, and the functional fragment is a polynucleotide encoding a transcription factor with a sequence identity of not less than 75% to the sequence of SEQ ID NO.19; or

The functional protein is a transcription factor functional protein with a sequence identity of not less than 85% to SEQ ID NO.18;

Preferably, the functional fragment is a polynucleotide encoding a functional protein, and the functional fragment is a polynucleotide encoding a transcription factor with a sequence identity of not less than 85% to SEQ ID NO.19; or

The functional protein is a transcription factor functional protein with a sequence identity of not less than 95% to SEQ ID NO.18;

More preferably, the functional fragment is a polynucleotide encoding a functional protein, and the functional fragment is a polynucleotide encoding a transcription factor with a sequence identity of not less than 95% to SEQ ID NO.19; or

The functional protein is a transcription factor functional protein with a sequence identity of not less than 99% to SEQ ID NO.18.

7. The recombinant oncolytic virus according to claim 1, wherein the expression system for the functional fragments promoting the expression of transcription factors is constructed under the same expression vector or is expressed using different expression vectors, respectively.

8. The recombinant oncolytic virus according to any one of claims 1-7, wherein the recombinant oncolytic virus comprises a selective replicable recombinant oncolytic virus.

9. The recombinant oncolytic virus according to claim 8, wherein the selected replicable recombinant oncolytic virus is derived from adenovirus, poxvirus, herpes simplex virus, measles virus, Semliki Forest virus, vesicular stomatitis virus, poliovirus, retrovirus, reovirus, Seneca Valley virus, echovirus, coxsackievirus, Newcastle disease virus or Maraba virus that have oncolytic activity.

10. A method for promoting reprogramming/trans-differentiation of tumor cells into non-tumorigenic cells by an oncolytic virus, wherein the method comprises the following steps:
Contacting the recombinant oncolytic virus according to any one of claims 1-9 with tumor cells, thereby reprogramming/trans-differentiating the tumor cells into non-tumorigenic cells.

11. A composition for treating cancer, wherein the composition comprises:

(A) the recombinant oncolytic virus according to any one of claims 1-9;
(B) a pharmaceutically acceptable excipient.

12. The composition according to claim 11, wherein the composition further comprises:

(C) an anti-tumor drug;

The anti-tumor drug includes one or both of temozolomide and bevacizumab.

13. Use of the recombinant oncolytic virus according to any one of claims 1-9 in the preparation of a medicament for treating tumors.

14. The use according to claim 13, wherein in the use, the recombinant oncolytic virus is formulated as a therapeutic agent for administration through the site within or near the tumor, and the administration method of the therapeutic agent includes injection delivery, intraperitoneal delivery, subarachnoid delivery, or intravenous delivery.

15. The use according to claim 13, wherein in the use, the recombinant oncolytic virus is formulated as a therapeutic agent that is administered through a site within or near the tumor, and the administration method of the therapeutic agent includes one or more of hydrogel delivery, convection-enhanced delivery and Ommaya reservoir delivery.

16. The use according to claim 13, wherein the tumor comprises: glioblastoma, neuroblastoma, chordoma, meningioma, teratoma, spinal cord tumor, breast cancer, head and neck tumor, renal cancer, melanoma, lung cancer, esophageal cancer, colon cancer, rectal cancer, brain cancer, liver cancer, bone cancer, choriocarcinoma, gastrinoma, pheochromocytoma, prolactinoma, cholangiocarcinoma, bladder cancer, ureter cancer, glioma, osteochondroma, chondrosarcoma, Ewing's sarcoma, fibrosarcoma, cervical cancer, gallbladder cancer, eye cancer, Kaposi's sarcoma, prostate cancer, testicular cancer, squamous cell carcinoma of the skin, mesothelioma, ovarian cancer, pancreatic neuroendocrine tumor, glucagonoma, pancreatic cancer, pituitary carcinoma, soft tissue sarcoma, retinoblastoma, small intestine cancer, gastric cancer, thymic cancer, trophoblastic cancer, endometrial cancer, vaginal cancer, vulvar cancer, insulinoma, hematological cancer, peritoneal cancer, or pleural cancer.

17. The use according to claim 13, wherein the tumor comprises: glioma, astrocytoma, astroblastoma, medulloblastoma, schwannoma, or brain metastasis tumors.

Fig. 1A

Fig. 1B

Fig. 2A

Fig. 2B

Fig. 3A

Fig. 3A-2

Fig. 3B

Fig. 3C

Fig. 4

Fig. 4-2

Fig. 5

Fig. 6

## INTERNATIONAL SEARCH REPORT

| | International application No. |
|---|---|
| | **PCT/CN2023/093135** |

**A. CLASSIFICATION OF SUBJECT MATTER**

C12N 7/01(2006.01)i; A61K 45/00(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNTXT, DWPI, USTXT, EPTXT, WOTXT , ENTXT, CNKI, 万方数据库, WANFANG DATABASE, WEB OF SCIENCE, PUBMED: 溶瘤, 病毒, 重组, 转录因子, 肿瘤, recombine, oncolytic, virus, transcription factor, NeuroD1, Brn2, Ascl1, Ngn2; GenBank+EMBL+中国专利生物序列检索系统: 对SEQ ID NOs: 3, 4, 7, 8, 11, 12, 15, 16, 18, 19的序列检索, GenBank+EMBL +China Patent Biological Sequence Search System: search for SEQ ID NOs: 3, 4, 7, 8, 11, 12, 15, 16, 18, 19

**C. DOCUMENTS CONSIDERED TO BE RELEVANT**

| Category* | Citation of document, with indication, where appropriate, of the relevant passages | Relevant to claim No. |
|---|---|---|
| Y | CN 105177045 A (YAN, Yang) 23 December 2015 (2015-12-23)<br>  see claims 1-5 | 1-9, 11-17 |
| Y | CN 113512565 A (THE PENN STATE RESEARCH FOUNDATION) 19 October 2021 (2021-10-19)<br>  see claims 1-20 and SEQ ID NOs:1 and 9 | 1-5, 7-9, 11-17 |
| Y | CN 112245592 A (WAYNE STATE UNIVERSITY et al.) 22 January 2021 (2021-01-22)<br>  see claims 1-3, description paragraph 80 and SEQ ID NOs: 91, 94, 96 and 30 | 1-9, 11-17 |
| Y | CN 107148471 A (TONGJI UNIVERSITY et al.) 08 September 2017 (2017-09-08)<br>  see claims 1-25 and SEQ ID NO: 1 | 1-5, 7-9, 11-17 |
| Y | CN 111770998 A (I PEACE, INC. et al.) 13 October 2020 (2020-10-13)<br>  see claims 1-12 and SEQ ID NOs: 3 and 1 | 1-5, 7-9, 11-17 |
| A | WO 2021194180 A1 (CURIGIN CO., LTD.) 30 September 2021 (2021-09-30)<br>  see abstract | 1-9, 11-17 |

☑ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **01 September 2023** | **11 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/093135** |

### C.    DOCUMENTS CONSIDERED TO BE RELEVANT

| Category* | Citation of document, with indication, where appropriate, of the relevant passages | Relevant to claim No. |
| --- | --- | --- |
| A | WO 2022052964 A1 (NEURAGEN BIOTHERAPEUTICS SUZHOU CO., LTD.) 17 March 2022 (2022-03-17)<br>        see abstract | 1-9, 11-17 |
| Y | 王晓鹏 等 (WANG, Xiaopeng et al.). "转录因子ZNF191腺病毒表达载体的构建及鉴定 (Construction and Identification of the Recombinant Adenovirus Expressing the Transcription Factor ZNF191)"<br>重庆医科大学学报 (*Journal of Chongqing Medical University*),<br>31 October 2011 (2011-10-31),<br>        pages 1215-1217, see abstract | 1-9, 11-17 |
| A | Carey A. Stuart et al. "RNA Polymerase Mutations Selected during Experimental Evolution Enhance Replication of a Hybrid Vaccinia Virus with an Intermediate Transcription Factor Subunit Replaced by the Myxoma Virus Ortholog"<br>*Journal of Virology*, 31 October 2018 (2018-10-31),<br>        pages e01089-18, see abstract | 1-9, 11-17 |

Form PCT/ISA/210 (second sheet) (July 2022)

**INTERNATIONAL SEARCH REPORT**

| International application No. |
| --- |
| **PCT/CN2023/093135** |

**Box No. I**       **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a. ☑ forming part of the international application as filed.

     b. ☐ furnished subsequent to the international filing date for the purposes of international search (Rule 13*ter*.1(a)),

         ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2023/093135**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **10**
because they relate to subject matter not required to be searched by this Authority, namely:

Said claim relates to a method for treatment of a disease in a living human or animal body, which falls within the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

<table>
<tr><td colspan="2">INTERNATIONAL SEARCH REPORT<br>Information on patent family members</td><td colspan="2">International application No.<br><br>**PCT/CN2023/093135**</td></tr>
</table>

| Patent document cited in search report | | | Publication date (day/month/year) | Patent family member(s) | | | Publication date (day/month/year) |
|---|---|---|---|---|---|---|---|
| CN | 105177045 | A | 23 December 2015 | None | | | |
| CN | 113512565 | A | 19 October 2021 | None | | | |
| CN | 112245592 | A | 22 January 2021 | None | | | |
| CN | 107148471 | A | 08 September 2017 | WO | 2016026438 | A1 | 25 February 2016 |
| | | | | US | 2022213502 | A1 | 07 July 2022 |
| | | | | US | 2017268021 | A1 | 21 September 2017 |
| | | | | US | 11261461 | B2 | 01 March 2022 |
| CN | 111770998 | A | 13 October 2020 | EP | 3719133 | A1 | 07 October 2020 |
| | | | | EP | 3719133 | A4 | 11 August 2021 |
| | | | | US | 2020392455 | A1 | 17 December 2020 |
| | | | | WO | 2019107354 | A1 | 06 June 2019 |
| | | | | JP | 6783954 | B2 | 11 November 2020 |
| | | | | JPWO | 2019107354 | A1 | 04 February 2021 |
| | | | | JP | 2020188814 | A | 26 November 2020 |
| | | | | JP | 7068405 | B2 | 16 May 2022 |
| WO | 2021194180 | A1 | 30 September 2021 | None | | | |
| WO | 2022052964 | A1 | 17 March 2022 | KR | 20230082024 | A | 08 June 2023 |
| | | | | EP | 4212624 | A1 | 19 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning: A Laboratory Manual. Cold Spring Harbor Laboratory Press, 1989

- **VIERBUCHEN, T et al.** Direct conversion of fibroblasts to functional neurons by defined factors. *Nature*, 2010, vol. 463, 1035-1041